(19) Europäisches Patentamt
European Patent Office
Office européen des brevets

(11) Veröffentlichungsnummer: **0 035 161**
**B1**

(12) # EUROPÄISCHE PATENTSCHRIFT

(45) Veröffentlichungstag der Patentschrift: **23.11.83**

(51) Int. Cl.³: **C 07 D 501/20**, A 61 K 31/545 // C07D417/12, C07D277/20

(21) Anmeldenummer: **81101107.1**

(22) Anmeldetag: **17.02.81**

(54) **Cephalosporine, Verfahren zu ihrer Herstellung und diese Verbindungen enthaltende Arzneimittel.**

(30) Priorität: **29.02.80 DE 3007685**

(43) Veröffentlichungstag der Anmeldung: **09.09.81 Patentblatt 81/36**

(45) Bekanntmachung des Hinweises auf die Patenterteilung: **23.11.83 Patentblatt 83/47**

(84) Benannte Vertragsstaaten: **AT BE CH DE FR GB IT LI LU NL SE**

(56) Entgegenhaltungen:
**EP - A - 0 006 532**
**EP - A - 0 021 176**
**EP - A - 0 022 494**
**FR - A - 2 418 800**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Wetzel, Bernd, Dr. Dipl.-Chem., Kapellenweg 21, D-7950 Biberach 1 (DE)**
Erfinder: **Woitun, Eberhard, Dr. Dipl.-Chem., Stecherweg 17, D-7950 Biberach 1 (DE)**
Erfinder: **Reuter, W., Dr. Dipl.-Chem., Nelkenweg 10, D-7951 Laupertshausen (DE)**
Erfinder: **Maier, Roland, Dr. Dipl.-Chem., Bodelschwinghstrasse 39, D-7950 Biberach 1 (DE)**
Erfinder: **Lechner, Uwe, Dr., Panoramastrasse 12, D-7951 Ummendorf (DE)**
Erfinder: **Goeth, Hanns, Dr., Oberer Bühl 6, D-7950 Biberach 1 (DE)**

**EP 0 035 161 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Cephalosporine, Verfahren zu ihrer Herstellung und diese Verbindungen enthaltende Arzneimittel

Die Erfindung betrifft neue Cephalosporine der allgemeinen Formel I:

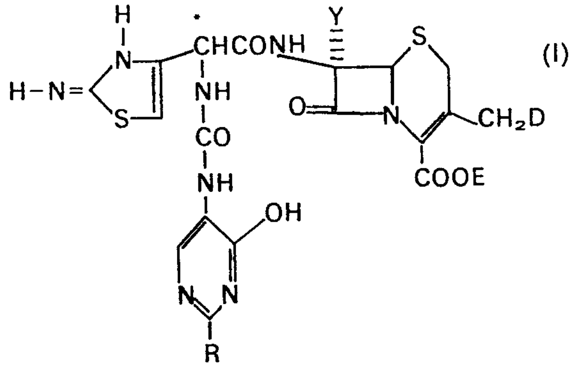

(I)

bzw. deren mögliche Tautomere, ihre physiologisch verträglichen Salze mit anorganischen oder organischen Basen, Verfahren zur Herstellung dieser Verbindungen und diese Verbindungen enthaltende Arzneimittel.

Durch die EP-A Nr. 0006532 und die FR-A Nr. 2418800 sind strukturell ähnliche Cephalosporine bekannt, die jedoch bezüglich ihrer Wirkungen insbesondere gegenüber wichtigen Problemkeimen wie *Pseudomonas aeruginosa* noch nicht ganz befriedigten.

In der obigen allgemeinen Formel I bedeuten

Y, das in der α-Konfiguration steht, ein Wasserstoffatom oder die Methoxygruppe,

D die Acetoxygruppe oder die Gruppe SHet, wobei Het die 1-Methyltetrazol-5-yl oder 2-Methyl-1,3,4-thiadiazol-5-ylgruppe darstellt,

E ein Wasserstoffatom oder ein Alkali- oder Erdalkalimetallion oder eine *in vitro* oder *in vivo* leicht abspaltbare Schutzgruppe darstellen, und

R die folgenden Gruppen: die Cyclopropylgruppe oder eine Gruppe der allgemeinen Formel $-NHR_1$, worin $R_1$ die Methyl-, Äthyl-, Propyl-, Isopropyl-, Cyclopentyl- oder Cyclohexylgruppe darstellt, oder R eine Gruppe der allgemeinen Formel $-NH-Z-X$, bei der $-NH-Z-$ eine Gruppe der Formeln

$$-\underset{H}{\underset{|}{N}}-CH_2-CH_2- \text{ oder } -\underset{H}{\underset{|}{N}}-(CH_2)_3-$$

darstellt, und

X die Hydroxy-, Methoxy-, Aminocarbonyl- oder Aminosulfonylgruppe bedeutet oder in der

$$-\underset{H}{\underset{|}{N}}-Z-X$$

die 4'-Hydroxycyclohexylaminogruppe bedeutet, R kann auch eine Gruppe der allgemeinen Formel:

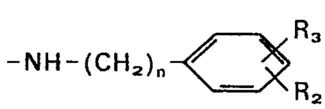

bedeuten, in der n=0 oder 1 ist und einer oder zwei der Reste $R_2$ und $R_3$ Wasserstoffatome, Chloratome, Methyl-, Hydroxy-, Acetylamino-, Methylsulfinyl-, Methylsulfonyl-, Aminocarbonyl-, Methylaminocarbonyl-, Aminosulfonyl-, Methylaminosulfonyl- oder Dimethylaminosulfonylgruppen oder Aminocarbonylaminogruppen darstellen.

Als Carboxylschutzgruppen für E kommen im Sinne der Erfindung solche in Frage, welche auch bisher schon auf dem Gebiet der Penicilline und Cephalosporine eingesetzt wurde, insbesondere esterbildende Gruppen, die durch Hydrogenolyse oder Hydrolyse oder andere Behandlungen unter milden Bedingungen entfernt werden können oder esterbildende Gruppen, welche leicht im lebenden Organismus abgespalten werden können.

Beispiele für *in vitro* leicht spaltbare Schutzgruppen sind z.B. die Benzyl-, Diphenylmethyl-Trityl-, t-Butyl-, die 2,2,2-Trichloräthyl- oder die Trimethylsilylgruppe. Beispiele für *in vivo* leicht spaltbare Schutzgruppen sind z.B. Alkanoyloxyalkylgruppen, wie z.B. die Acetoxymethyl-, Propionyloxymethyl-, 2-Acetoxyäthyl- oder Pivaloyloxymethylgruppe oder die Phthalidylgruppe. Bedeutet E ein Wasserstoffatom, so fallen unter den Anspruch auch pharmakologisch verträgliche Salze mit anorganischen oder organischen Basen, wie z.B. die Alkali- oder Erdalkalisalze, z.B. die Natrium-, Kalium-, Magnesium- oder Calciumsalze, die Ammoniumsalze, oder organische Aminsalze, z.B. solche mit Triäthylamin oder Dicyclohexylamin.

Das Sternchen an dem Kohlenstoffatom in der Verbindung der allgemeinen Formel I bedeutet ein Asymmetriezentrum.

Bevorzugt sind diejenigen Verbindungen der allgemeinen Formel I, worin

Y ein Wasserstoffatom oder die Methoxygruppe,

D eine Acetoxygruppe oder die Gruppe SHet bedeuten, wobei Het die 1-Methyltetrazol-5-yl- oder die 2-Methyl-1,3,4-thiadiazol-5-ylgruppe darstellt, und

R und E die obenangegebenen Bedeutungen haben.

Besonders bevorzugt sind diejenigen Verbindungen der allgemeinen Formel I, worin

Y ein Wasserstoffatom oder die Methoxygruppe,

D die Acetoxygruppe oder die Gruppe SHet, wobei Het die 1-Methyltetrazol-5-yl- oder 2-Methyl-1,3,4-thiadiazol-5-ylgruppe,

E ein Wasserstoffatom oder Natriumion bedeuten und R die folgenden Bedeutungen besitzt: die Cyclopropylgruppe oder eine Gruppe der allgemeinen Formel $NHR_1$, worin $R_1$ die Methyl-, Äthyl-, Propyl-, Isopropyl-, Cyclopentyl- oder Cyclohexylgruppe darstellt, oder worin R für eine Gruppe der allgemeinen Formel $NH-Z-X$ steht, bei der $NH-Z-$ eine Gruppe der Formeln

$$-\underset{H}{\underset{|}{N}}-CH_2-CH_2- \text{ oder } -\underset{H}{\underset{|}{N}}-(CH_2)_3-$$

darstellt, und X die Hydroxy-, Methoxy-, Aminocarbonyl- oder Aminosulfonylgruppe bedeutet oder in der

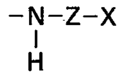

die 4'-Hydroxycyclohexylaminogruppe bedeutet.

Des weiteren ist eine besonders bevorzugte Bedeutung von R eine Gruppe der allgemeinen Formel:

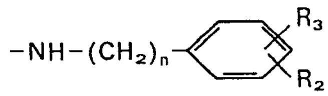

in der n=0 oder 1 und einer oder zwei der Reste $R_2$ und $R_3$ Wasserstoffatome, Chloratome, Methyl-, Hydroxy-, Acetylamino-, Methylsulfinyl-, Methylsulfonyl-, Aminocarbonyl-, Methylaminocarbonyl-, Aminosulfonyl-, Methylaminosulfonyl-, Dimethylaminosulfonyl- oder Aminocarbonylaminogruppen bedeuten.

Ganz besonders bevorzugte Reste der allgemeinen Formel

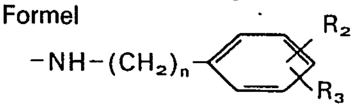

sind der p-Aminosulfonylanilino-, der p-Methylsulfinylanilino-, der p-Methylsulfonylanilino-, der m-Hydroxy-p-aminosulfonylanilino-, der m,p-Bis(aminocarbonyl)anilino-, der p-Aminosulfonylbenzylamino-, der m,p-Dihydroxybenzylamino- und der p-Hydroxybenzylaminorest.

Die Cephalosporinverbindungen der allgemeinen Formel I und die nachstehend beschriebenen Zwischenprodukte können in mehreren tautomeren Formen (nämlich bezüglich des Pyrimidinrings und der 2-Aminothiazolylgruppe) vorliegen. Es hängt besonders vom jeweiligen Lösungsmittel und von der Art der Substituenten R ab, welche der nachstehenden Formeln I, I', I'', I''' überwiegt:

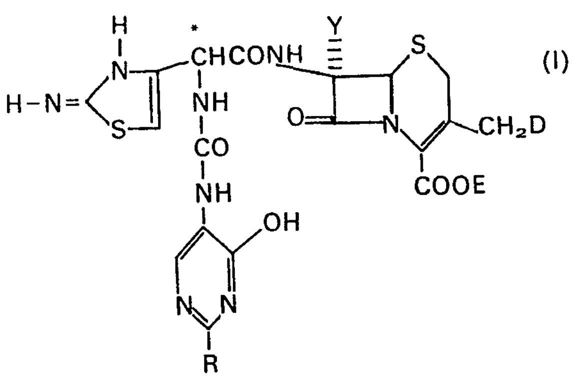

(I)

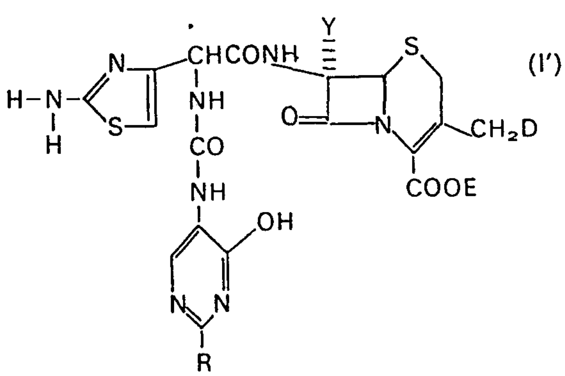

(I')

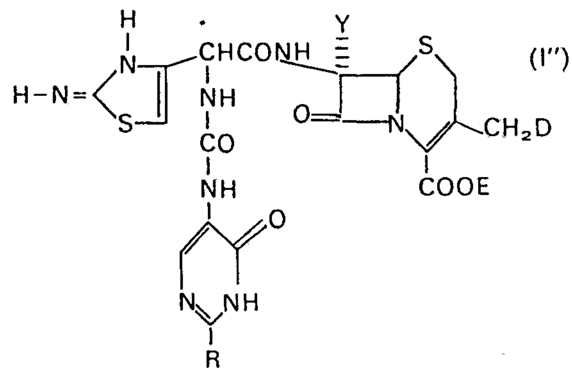

(I'')

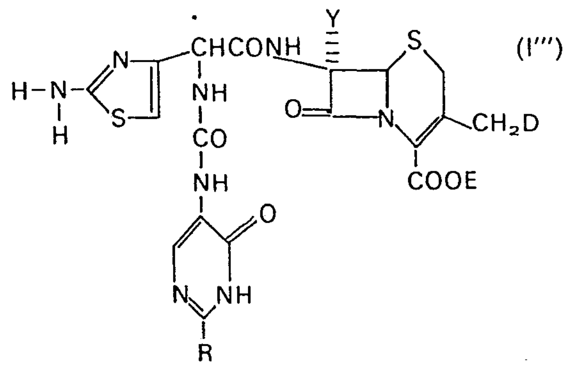

(I''')

Es versteht sich von selbst, dass die eingangs angegebenen Verbindungen der allgemeinen Formel I immer alle Tautomeren umfasst.

Die Verbindungen der allgemeinen Formel I können bezüglich des mit einem Sternchen gekennzeichneten Chiralitätszentrums C* in den beiden möglichen R- und S-Konfigurationen, jedoch auch als ein Gemisch dieser Konfigurationen vorliegen.

Es wurde gefunden, dass die Verbindungen der allgemeinen Formel I wertvolle pharmakologische Eigenschaften bei guter Verträglichkeit besitzen. Die erfindungsgemässen Wirkstoffe können daher zur Prophylaxe und Chemotherapie von lokalen und systemischen Infektionen in der Human- und Tiermedizin verwendet werden. Als Krankheiten, die durch die erfindungsgemässen Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen. Weiter können diese Verbindungen als Stoffe zur Konservierung von anorganischen oder organischen Materialien verwendet werden, besonders von organischen Materialien, wie Polymere, Schmiermittel, Farben, Fasern, Leder, Papier und Holz sowie Lebensmittel.

Dieses wird dadurch ermöglicht, dass die Verbindungen der allgemeinen Formel I sowohl *in vitro* als auch *in vivo* gegen schädliche Mikroorganismen, insbesondere gegen grampositive und gramnegative Bakterien und bakterienähnliche Mikroorganismen sehr stark wirken, wobei sie sich besonders durch ein breites Wirkungsspektrum auszeichnen.

Mit diesen Cephalosporinderivaten können beispielsweise lokale und/oder systemische Erkrankungen behandelt und/oder verhindert werden, die durch die folgenden Erreger oder durch Gemische der folgenden Erreger verursacht werden:
*Micrococcaceae*, wie Staphylokokken;
*Lactobacteriaceae*, wie Streptokokken;
*Neisseriaceae*, wie Neisserien;
*Corynebacteriaceae*, wie Corynebakterien;
*Enterobacteriaceae*, wie *Escherichiae*-Bakterien der *Coli*-Gruppe;
*Klebsiella*-Bakterien, z.B. *Klebsiella pneumoniae*;
*Proteae*-Bakterien der *Proteus*-Gruppe z.B. *Proteus vulgaris*;
*Salmonella*-Bakterien, z.B. *Salmonella thyphimurium*;
*Shigella*-Bakterien, z.B. *Shigella dysenteriae*;
*Pseudomonas*-Bakterien, z.B. *Pseudomonas aeruginosa*;
*Aeromonas*-Bakterien, z.B. *Aeromonas lique faciens*;
*Spirillaceae*, wie *Vibrio*-Bakterien, z.B. *Vibrio cholerae*;
*Parvobacteriaceae* oder *Brucellaceae*, wie *Pasteurella*-Bakterien;
*Brucella*-Bakterien, z.B. *Brucella abortus*;
*Haemophilus*-Bakterien, z.B. *Haemophilus influenzae*;
*Bordetella*-Bakterien, z.B. *Bordetella pertussis*;
*Moraxella*-Bakterien, z.B. *Moraxella lacunata*;
*Bacteroidaceae*, wie *Bacteroides*-Bakterien;
*Fusiforme*-Bakterien, z.B. *Fusobacterium fusiforme*;
*Sphaerophorus*-Bakterien, z.B. *Sphaerophorus necrophorus*;
*Bacillaceae*, wie aerobe Sporenbildner, z.B. *Bacillus anthracis*;
anaerobe Sporenbildner-Chlostridien, z.B. *Clostridium perfringens*;
*Spirochaetaceae*, wie *Borrelia*-Bakterien;
*Treponema*-Bakterien, z.B. *Treponema pallidum*;
*Leptospira*-Bakterien, wie *Leptospira interrogans*.

Die obige Aufzählung von Erregern ist lediglich beispielhaft und keineswegs beschränkend aufzufassen.

Die Verbindungen der allgemeinen Formel I können wie folgt hergestellt werden:

1. Die Verbindungen der allgemeinen Formel I können durch Umsetzung eines 7-Aminocephalosporansäurederivates der allgemeinen Formel II:

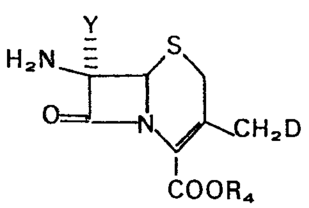

(II)

in der D wie oben definiert ist und $R_4$ die tert.-Butyl-, Benzyl-, Diphenylmethyl-, Trimethylsilyl- oder 2,2,2-Trichloräthyl-, insbesondere aber die Trimethylsilyl- oder die Diphenylmethylgruppe darstellt, mit Ureidocarbonsäuren der allgemeinen Formel III:

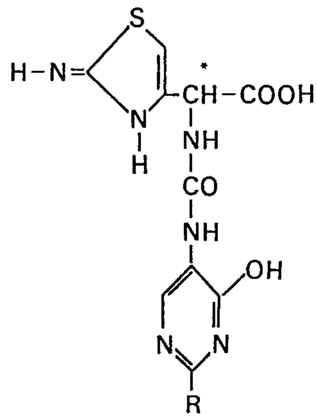

(III)

in der R die vorstehende Bedeutung hat, oder ihren Salzen oder reaktiven Derivaten unter Bildung eines Zwischenproduktes der allgemeinen Formel IV:

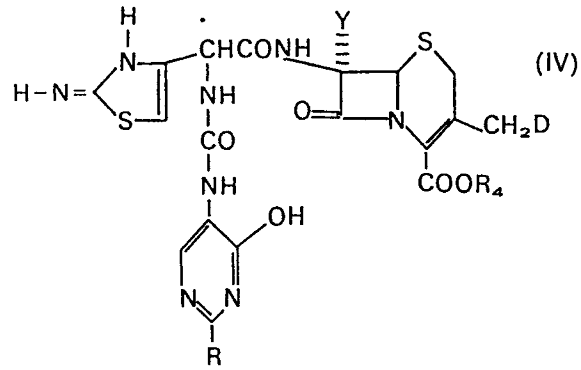

(IV)

hergestellt werden.

Als reaktive Derivate der Ureidocarbonsäuren der allgemeinen Formel III kommen beispielsweise deren Säureanhydride wie z.B. die, die sich von Chlorameisensäureestern, z.B. Chlorameisensäureäthyl- oder -isobutylester, ableiten, oder deren reaktive Ester, wie der p-Nitrophenylester oder der N-Hydroxysuccinimidester, oder deren reaktive Amide, wie das N-Carbonylimidazol, aber auch deren Säurehalogenide, wie das entsprechende Säurechlorid oder deren Säureazide in Frage. Prinzipiell können jedoch alle Verknüpfungsmethoden, wie sie aus der β-Lactamchemie bekannt sind, verwendet werden.

Die Ureidocarbonsäure, ihre Salze oder ihre reaktiven Derivate werden mit den 7-Aminocephalosporansäurederivaten in einem Lösungsmittel bei Temperaturen zwischen −40 und +40°C, gegebenenfalls in Gegenwart einer Base, umgesetzt. Wird z.B. ein Anhydrid der Ureidocarbonsäure, beispielsweise das Anhydrid mit dem Äthylchloroformiat, eingesetzt, so wird die Reaktion unter Kühlung, beispielsweise bei −10 bis +10°C in einem Lösungsmittel, wie Aceton, Tetrahydrofuran, Dimethylformamid, Chloroform, Dichlormethan, Hexametapol oder in einem Gemisch dieser Lösungsmittel, durchgeführt. Setzt man beispielsweise einen N-Hydroxysuccinimidester der Ureidocarbonsäure mit Derivaten der allgemeinen Formel II um, so wird die Reaktion vorzugsweise bei 0 und 20°C in Anwesenheit einer Base, wie z.B. Triäthylamin, in einem Lösungsmittel, wie Dimethylformamid, Dichlormethan oder Dioxan, oder in einem Gemisch solcher Lösungsmittel durchgeführt.

Die Umsetzung einer Ureidocarbonsäure der allgemeinen Formel III selbst oder ihrer Salze mit Verbindungen der allgemeinen Formel II erfolgt vorteilhafterweise in Gegenwart eines Kondensationsmittels, z.B. in Gegenwart von N,N'-Dicyclohexylcarbodiimid. Dieses direkte Acylierungsverfahren ist besonders dann bevorzugt, wenn A ein Wasserstoffatom darstellt.

Das so erhaltene Zwischenprodukt der allgemeinen Formel IV wird, wenn $R_4$ beispielsweise Diphenylmethyl bedeutet, mit Trifluoressigsäure und Anisol in bekannter Weise zu den Verbindungen der allgemeinen Formel I umgesetzt. Bedeutet $R_4$ beispielsweise Trimethylsilyl, so kann die Abspaltung der Silylschutzgruppe in üblicher Weise durch wässerige Hydrolyse erfolgen.

2. Die Verbindungen der allgemeinen Formel I, I', I'' oder bzw. I''' werden auch erhalten durch Umsetzung von 7-Aminocephalosporansäuren der allgemeinen Formel V oder ihrer Salze mit anorganischen oder organischen Säuren:

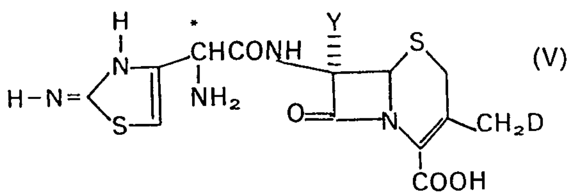

(V)

in der D und Y wie oben definiert sind, mit einem Pyrimidinderivat der allgemeinen Formel VI:

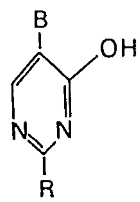

(VI)

in der R wie oben definiert ist und B die Gruppe −NCO oder ein reaktives Derivat der Gruppe −NHCOOH bedeutet, wie z.B. die Gruppen

−NHCOCl, −NHCOBr

oder −NH−COO− 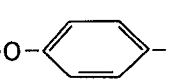 −$NO_2$,

wobei die Gruppe NHCOCl besonders bevorzugt ist. Es können auch Gemische von solchen Pyrimidinderivaten der allgemeinen Formel VI verwendet werden, in der B teils die eine und teils die andere der vorstehend genannten Bedeutungen besitzt, z.B. die Gruppen −NCO und −NH−COCl gleichzeitig nebeneinander.

Die Reaktion wird bevorzugt in beliebigen Mischungen von Wasser mit solchen organischen Lösungsmitteln, die mit Wasser mischbar sind, wie Ketonen, z.B. Aceton, cyclische Äther, z.B. Tetrahydrofuran oder Dioxan, Nitrilen, z.B. Acetonitril, Formamiden, z.B. Dimethylformamid, Dimethylsulfoxid oder Alkoholen z.B. Isopropanol oder in Hexametapol durchgeführt. Besonders bevorzugt ist ein Gemisch aus Tetrahydrofuran und Wasser.

Dabei hält man den pH-Wert der Reaktionsmischung durch Zusatz von Basen oder Verwendung von Pufferlösungen in einem pH-Bereich von etwa 2,0 bis 9,0, vorzugsweise zwischen pH-Wert 6,5 und 8,0.

3. Die Verbindungen der allgemeinen Formel I, I', I'' oder I''', in der E für Wasserstoff steht und D für den Rest SHet, können durch Umsetzung einer Verbindung der allgemeinen Formel VII:

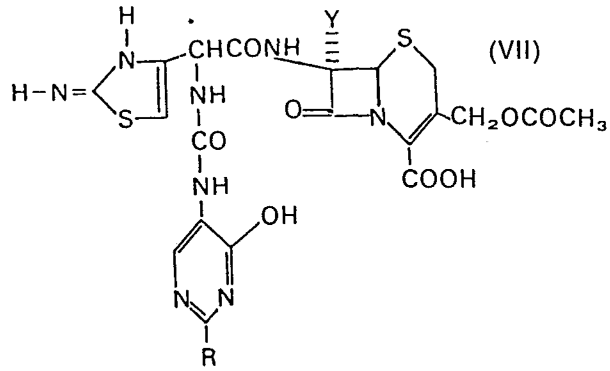

(VII)

in der R und Y die oben angegebenen Bedeutungen haben, mit einer Verbindung der allgemeinen Formel VIII:

Het−S−M (VIII)

in der Het die oben angegebenen Bedeutungen hat und M für ein Wasserstoffatom oder ein Alkalimetall- oder ein Erdalkalimetallatom steht, hergestellt werden. Dazu wird z.B. eine Verbindung der Formel VII mit beispielsweise 1-Methyl-5-mercapto-1,2,3,4-tetrazol in einem Lösungsmittel, z.B. Wasser, Methanol, Äthanol, Aceton, Methyläthylketon, Tetrahydrofuran, Acetonitril, Essigsäureäthylester, Dimethoxyäthan, Dimethylformamid, Dimethylsulfoxid, Chloroform oder einem Gemisch dieser Lösungsmittel umgesetzt. Vorzugsweise wird ein stark polares Lösungsmittel, wie Wasser verwendet: In diesem Fall wird der pH-Wert der Reaktionslösung vorteilhafterweise auf 2 bis 10 und insbesondere auf 4 bis 8 gehalten. Der gewünschte pH-Wert kann durch Zugabe einer Pufferlösung, wie Natriumphosphat, eingestellt werden. Die Reaktionsbedingungen unterliegen keinen besonderen Beschränkungen. Normalerweise wird die Umsetzung bei einer Temperatur im Bereich von 0 bis 100°C während einer Zeitdauer von einigen Stunden durchgeführt.

4. Eine Verbindung der allgemeinen Formel I, I', I'' oder I''', in der Y die Methoxygruppe darstellt, kann dadurch erhalten werden, dass man eine Verbindung der allgemeinen Formel I, I', I'' oder I''', in der Y ein Wasserstoffatom bedeutet, in Anwesenheit von Methanol mit einem Alkalimetallmethylat der allgemeinen Formel $M^+OCH_3^-$, worin $M^+$ ein Alkalimetallion bedeutet, und dann mit einem Halogenierungsmittel umsetzt. Dazu wird ein Cephalosporin der allgemeinen Formel I, in der Y ein Wasserstoffatom darstellt, in einem inerten Lösungsmittel, z.B. Tetrahydrofuran, Dioxan, Äthylenglycoldimethyläther, Methylenchlorid, Chloroform, Dimethylformamid oder Methanol oder in einem Gemisch von zweien dieser Lösungsmittel

aufgelöst oder suspendiert. Zu der so erhaltenen Lösung oder Suspension gibt man ein Alkalimetallmethylat zusammen mit Methanol.

Das so erhaltene Gemisch wird zur Reaktion gebracht und das Reaktionsgemisch wird dann mit einem Halogenierungsmittel umgesetzt. Bei dieser Reaktion verwendet man Methanol im Überschuss und die Menge des Alkalimetallmethylats beträgt, vorzugsweise 2 bis 8 Äquivalente pro Äquivalent des verwendeten Cephalosporins. Der Ausdruck im Überschuss bedeutet eine Menge von mehr als einem Äquivalent pro Äquivalent des Cephalosporins. Alle Reaktionen werden bei −120 bis −10°C und vorzugsweise −100 bis −50°C durchgeführt. Eine Reaktionszeit von 5 bis 60 min reicht aus. Die Reaktion wird durch Ansäuern des Reaktionssystems abgebrochen.

Das bei diesem Verfahren eingesetzte Halogenierungsmittel ist allgemein als Quelle für positive Halogenatome, z.B. $Cl^+$ oder $Br^+$, $J^+$ bekannt. Beispiele solcher Halogenierungsmittel sind Halogen, wie Chlor oder Brom; N-Halogenimide, wie N-Chlorsuccinimid oder N-Bromsuccinimid; N-Halogenamide, wie N-Chloracetamid oder N-Bromacetamid; N-Halogensulfonamide, wie N-Chlorbensulfonamid oder N-Chlor-p-toluolsulfonamid; 1-Halogenbenzotriazole; 1-Halogentriazine, organische Hypohalogenite, wie tert.-Butylhypochlorit oder tert.-Butylhypojodit; Halogenhydantoine, wie N,N-Dibromhydantoin. Unter diesen Halogenierungsmitteln ist tert.-Butylhypochlorit bevorzugt. Das Halogenierungsmittel wird in einer Menge eingesetzt, welche ausreicht, eine zur Menge des Cephalosporins der allgemeinen Formel I äquivalente Menge positiven Halogens abzugeben. Geeignete Säuren für das Abbrechen der Reaktion sind solche, welche bei Zusatz zum kalten Reaktionsgemisch nicht zu einer Verfestigung des Reaktionsgemisches oder zum Gefrieren des Reaktionsgemisches zu einer schweren viskosen Masse führen. Beispiele geeigneter Säuren sind 98%ige Ameisensäure, Eisessig, Trichloressigsäure und Methansulfonsäure. Nach dem Abbrechen der Reaktion wird das überschüssige Halogenierungsmittel durch Behandlung mit einem Reduktionsmittel, z.B. Trialkylphosphit oder Natriumthiosulfat entfernt.

Die Verbindungen der allgemeinen Formel I, in der E ein Natrium- oder Kaliumkation darstellt, werden durch Umsetzung der entsprechenden freien Säure der Verbindungen der allgemeinen Formel I, in der E ein Wasserstoffatom darstellt, mit dem entsprechenden salzbildenden Ion hergestellt. Hierzu eignet sich zum Beispiel die in der Chemie der Penicilline und Cephalosporine übliche Umsetzung mit Natriumäthylhexanoat, oder die Umsetzung mit Natriumhydrogencarbonat und anschliessende Gefriertrocknung. Die Cephalosporinantibiotika der allgemeinen Formel I, in der E ein Wasserstoffatom bedeutet, können auf bekannte Weise in die Acyloxyalkylester, worin E z.B. einen Pivaloyloxymethylrest

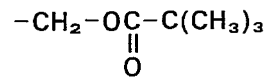

darstellt, überführt werden, indem man ein Alkalisalz der Cephalosporincarbonsäure, beispielsweise ein Natrium- oder Kaliumsalz, mit einem Pivaloyloxymethylhalogenid der Formel:

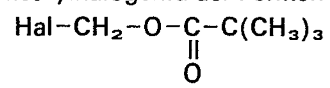

worin Hal für ein Chlor-, Brom- oder Jodatom steht, umsetzt. Weitere geeignete Acyloxyalkylhalogenide sind z.B. Chlormethylacetat, Brommethylpropionat oder 1-Bromäthylacetat.

Bei Verwendung der entsprechenden Ausgangsverbindungen ist es möglich, die Verbindungen der allgemeinen Formel I in Form der Racemate oder in Form der einzelnen Isomeren herzustellen. Wenn das Endprodukt in der D,L-Form anfällt, gelingt es, die reinen D- und L-Diastereoisomeren durch präparative Flüssigkeitschromatographie (HPLC) herzustellen. Die Erfindung betrifft auch die Racemate und die Isomeren.

Die Ureidocarbonsäurederivate der allgemeinen Formel III werden erhalten, indem man die Aminosäure der allgemeinen Formel IX oder ihre Salze mit Säuren, z.B. mit $CF_3COOH$,

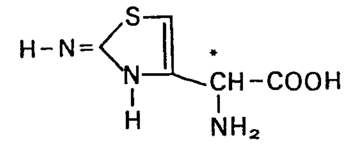

(IX)

mit einem Pyrimidinderivat der allgemeinen Formel VI:

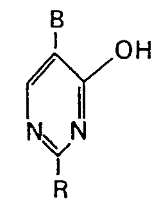

(VI)

in der R und B wie oben definiert sind, umsetzt.

Die Umsetzung erfolgt bei Temperaturen zwischen −20 und +40°C, vorzugsweise zwischen 0 und +20°C, in einem Lösungsmittel. Als Lösungsmittel können hierbei z.B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol oder Dimethylsulfoxid. Gegebenenfalls wird die Verwendung eines halogenwasserstoffbindenden Mittels notwendig, als solche eignen sich beispielsweise Trialkylamine, wie Triäthylamin oder anorganische Basen, wie verdünnte Natronlauge.

Derivate der allgemeinen Formel IX sind literaturbekannt und beispielsweise in den DE-OS Nrn. 2924296 und 2556736 beschrieben.

Die Ausgangsstoffe der allgemeinen Formel VI können beispielsweise durch Umsetzung der entsprechenden 5-Aminopyrimidine der allgemeinen Formel X:

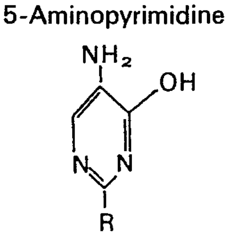

(X)

in der R wie oben definiert ist, mit Phosgen gewonnen werden. Diese Umsetzung erfolgt vorzugsweise in einem keine Hydroxylgruppen enthaltenden Lösungsmittel, wie Tetrahydrofuran, Methylenchlorid, Chloroform, Dimethoxyäthan oder Hexametapol bei Temperaturen zwischen −40 und +60°C, vorzugsweise zwischen −10 und +20°C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuss verwendet werden. Sollten die betreffenden Aminopyrimidine der allgemeinen Formel X in einem der erwähnten Lösungsmittel schwer löslich sein, so kann die Phosgenierung auch in heterogener Phase durchgeführt werden. Des weiteren können in einer besonders bevorzugten Ausführungsform die Aminopyrimidine der allgemeinen Formel X durch Behandlung mit einem Silylierungsmittel, wie Hexamethyldisilazan, Trimethylchlorsilan/Triäthylamin, Trimethylsilyldiäthylamin oder N,O-Bistrimethylsilylacetamid in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel VI reagiert, wobei bevorzugt ohne Basenzusatz gearbeitet wird. Je nach der Art des Lösungsmittels, der Höhe der Temperatur, der Menge und Art einer eventuell eingesetzten Base entsteht entweder überwiegend das entsprechende Isocyanat oder Carbaminsäurehalogenid oder ein Gemisch dieser beiden Verbindungen. Je nach den Bedingungen liegt das Isocyanat der allgemeinen Formel VI auch teilweise oder ganz als ein den Isocyanaten isomeres Dihydrooxazolo[5,4-d]pyrimidin der allgemeinen Formel VIa:

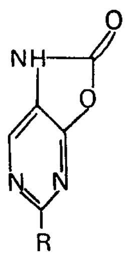

(VIa)

oder als ein je nach Art des Substituenten R ein oder mehrfach silyliertes Analoges vor.

Die durch Phosgenierung entstandenen Ausgangsprodukte der allgemeinen Formel VI bzw. deren Gemische oder deren Silylprodukte sind in den obenerwähnten Lösungsmitteln im allgemeinen gut löslich und können, nach Entfernung des überschüssigen Phosgens, ohne weitere Reinigung mit den entsprechenden Cephalosporinderivaten der allgemeinen Formel V direkt umgesetzt werden. Es ist jedoch auch möglich, das Zwischenprodukt der allgemeinen Formel VIa zu isolieren, es gegebenfalls mit einem protischen Lösungsmittel zu entsilylieren, z.B. mit Methanol oder Wasser, es erforderlichenfalls auf Grund seiner Löslichkeitseigenschaften zu reinigen und in der obendargestellten Weise umzusetzen.

Synthesen für 2-substituierte 5-Amino-4-hydroxypyrimidine der allgemeinen Formel X sind literaturbekannt und werden in der DE-OS Nr. 2928344 beschrieben.

Die Synthese von Ausgangsprodukten der allgemeinen Formel V ist literaturbekannt. Dazu wird ein Cephalosporinderivat der allgemeinen Formel II mit einer an der Aminogruppe geschützten Aminosäure der allgemeinen Formel IX unter den in der Cephalosporinchemie üblichen Bedingungen umgesetzt und anschliessend die Schutzgruppen wie üblich abgespalten (vgl. z.B. „J. Antibiotics" 1980, 1022 und DE-OS Nr. 2924296). Die 7-Aminocephalosporansäurederivate der allgemeinen Formel II sind literaturbekannt. Die Ausgangsverbindungen der allgemeinen Formel VII lassen sich aus den Ureidocarbonsäuren der allgemeinen Formel III, worin A Wasserstoff ist, und den 7-Aminocephalosporansäurederivaten der allgemeinen Formel II herstellen.

Wie bereits dargelegt, sind besonders gut wirksame Verbindungen der allgemeinen Formel I solche, in denen E für Wasserstoff steht. Es sollen besonders die folgenden Verbindungen genannt werden:

1. Natrium-7β-{D,L-α-[(2-cyclopropyl-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

2. Natrium-7β-{D,L-α-[(2-isopropylamino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

3. Natrium-7β-{D,L-α-[(2-propylamino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

4. Natrium-7β-{D,L-α-[(2-cyclopentylamino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

5. Natrium-7β-{D,L-α-[(2-(2'-hydroxyäthylamino)-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

6. Natrium-7β-{D,L-α-[(2-(3'-hydroxypropylamino)-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

7. Natrium-7α-methoxy-7β-{-D,L-α-[(2-(3'-hydroxypropylamino)-4-hydroxy-5-pyrimidinyl)ureido]-(2.3-dihydro-2-imino-4-thiazolyl)-acetamido}-3-[(1-methyltetrazol-5-yl)thiomethyl]ceph-3-em-4-carboxylat

8. Natrium-7β-{D,L-α-[(2-(4'-hydroxycyclohexylamino)-4-hydroxy-5-pyrimidinyl)ureido]-(2.3-dihydro-2-imino-4-thiazolyl)acetamido}-3-

[(1-methyltetrazol-5-yl)thiomethyl]ceph-3-em-4-carboxylat

9. Natrium-7α-methoxy-7β-{D,L-α-[(2-(4'-hydroxycyclohexylamino)-4-hydroxy-5-pyrimidinyl)ureido]-(2.3-dihydro-2-imino-4-thiazolyl)acetamido}-3-[(1-methyltetrazol-5-yl)-thiomethyl]ceph-3-em-4-carboxylat

10. Natrium-7β-{D,L-α-[(2-(2'-aminosulfonyläthylamino)-4-hydroxy-5-pyrimidinyl)ureido]-(2.3-dihydro-2-imino-4-thiazolyl)acetamido}-3-[(1-methyltetrazol-5-yl)thiomethyl]-ceph-3-em-4-carboxylat

11. Natrium-7β-{D,L-α-[(2-(3'-aminocarbonylpropylamino)-4-hydroxy-5-pyrimidinyl)-ureido]-(2.3-dihydro-2-imino-4-thiazolyl)acetamido}-3-[(1-methyltetrazol-5-yl)thiomethyl]-ceph-3-em-4-carboxylat

12. Natrium-7β-{D,L-α-[(2-(4'-hydroxycyclohexylamino)-4-hydroxy-5-pyrimidinyl)-ureido]-(2.3-dihydro-2-imino-4-thiazolyl)acetamido}-3-[(1-methyltetrazol-5-yl)thiomethyl]-ceph-3-em-4-carboxylat

13. Natrium-7β-{D,L-α-[(2-p-aminosulfonylanilino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

14. Natrium-7α-methoxy-7β-{D,L-α-[(2-p-aminosulfonylanilino-4-hydroxy-5-pyrimidinyl)-ureido]-(2.3-dihydro-2-imino-4-thiazolyl)acetamido}-3-[(1-methyltetrazol-5-yl)thiomethyl]-ceph-3-em-4-carboxylat

15. Natrium-7β-{D,L-α-[(2-p-aminosulfonylanilino-4-hydroxy-5-pyrimidinyl)ureido]-(2.3-dihydro-2-imino-4-thiazolyl)acetamido}-3-acetoxymethylceph-3-em-4-carboxylat

16. Natrium-7β-{D,L-α-[(2-p-aminosulfonylanilino-4-hydroxy-5-pyrimidinyl)ureido]-(2.3-dihydro-2-imino-4-thiazolyl)acetamido}-3-[(2-methyl-1.3.4-thiadiazol-5-yl)thiomethyl]ceph-3-em-4-carboxylat

17. Natrium-7β-{D,L-α-[(2-p-methylsulfinylanilino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

18. Natrium-7β-{D,L-α-[(2-p-methylsulfonylanilino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

19. Natrium-7β-{D,L-α-[(2-(m-hydroxy-p-aminosulfonylanilino)-4-hydroxy-5-pyrimidinyl)ureido]-(2.3-dihydro-2-imino-4-thiazolyl)-acetamido}-3-[(1-methyltetrazol-5-yl)thiomethyl]ceph-3-em-4-carboxylat

20. Natrium-7α-methoxy-7β-{D,L-α-[(2-(m-hydroxy-p-aminosulfonylanilino)-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

21. Natrium-7β-{D,L-α-[(2-(m,p-bisaminocarbonylanilino)-4-hydroxy-5-pyrimidinyl)ureido]-(2.3-dihydro-2-imino-4-thiazolyl)acetamido}-3-[(1-methyltetrazol-5-yl)-thiomethyl]ceph-3-em-4-carboxylat

22. Natrium-7β-{D,L-α-[(2-p-hydroxybenzylamino)-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

23. Natrium-7β-{D,L-α-[(2-(m,p-dihydroxybenzylamino)-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

24. Natrium-7β-{D,L-α-[(2-(p-aminosulfonylbenzylamino)-4-hydroxy-5-pyrimidinyl)-ureido]-(2.3-dihydro-2-imino-4-thiazolyl)acetamido}-3-[(1-methyltetrazol-5-yl)thiomethyl]-ceph-3-em-4-carboxylat

25. Natrium-7β-{D-α-[(2-p-aminosulfonylanilino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

26. Natrium-7β-{L-α-[(2-p-aminosulfonylanilino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

27. Natrium-7β-{D-α-[(2-p-hydroxybenzylamino)-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

28. Natrium-7β-{L-α-[(2-p-hydroxybenzylamino)-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat

Die Wirksamkeit der erfindungsgemässen β-Lactamantibiotika lässt sich durch folgende Untersuchungen beispielhaft demonstrieren:

1. In vitro-*Versuche*

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht: 128, 64, 32, 16, 8, 4, 2, 1, 0,5, 0,25, 0,12, 0,06, 0,03, 0,015 µg/ml.

Es wurde ein Nährboden folgender Zusammensetzung benutzt: 10 g Pepton, 8 g Fleischextraktoxoid, 3 g Natriumchlorid, 2 g sek.-Natriumphosphat werden mit dest. Wasser auf 100 ml aufgefüllt (pH-Wert 7,2 bis 7,4). Das Alter der Primärkulturen betrug etwa 20 h. Die Einstellung der Keimsuspension erfolgte am Photometer (nach Eppendorf) (Reagenzglasdurchmesser 14 mm, Filter 546 nm) anhand der Trübung einer Bariumsulfatvergleichssuspension, die durch eine Bariumsulfataufschwemmung erzeugt wurde, welche durch die Zugabe von 3,0 ml 1%ige Bariumchloridlösung in 97 ml 1%ige Schwefelsäure entstand. Nach der Einstellung wurden die

Testkeime im Verhältnis 1:1500 mit einer Kochsalzlösung weiter verdünnt.

16 mg der jeweiligen Substanz wurde in 10-ml-Messkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 h bei 37°C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

Als Testorganismen wurden benützt:

*Staphylococcus aureus* SG 511, *Escherichia coli* ATCC 11775, *Pseudomonas aeruginosa Hamburgensis und Pseudomonas aeruginosa* Walter, *Serratia marcescens* ATCC 13880, *Klebsiella pneumoniae* ATCC 10031 und BC 6, *Proteus mirabilis* BC 17; *Proteus rettgeri* BC 7, *Enterobacter cloacae* ATCC 13047, *Escherischia coli* R+TEM (β-Lactamaseträger), *Klebsiella pneumoniae* 1082 E (β-Lactamaseträger).

In der folgenden Tabelle 1 sind die ermittelten minimalen Hemmkonzentrationen (MIC) für typische Vertreter der erfindungsgemässen Verbindungen aufgeführt:

Es wurden z.B. folgende Natriumsalze von Verbindungen der allgemeinen Formel I mit folgenden Bedeutungen von R, D und Y untersucht:

| R | D | Y | Verbindung |
|---|---|---|---|
| 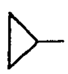 | 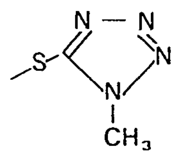 | H | A |
| $-NHC_3H_7$ | 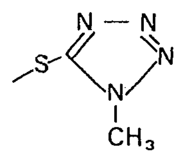 | H | B |
| $-NH(CH_2)_3OH$ | 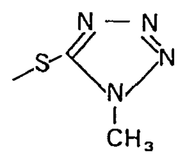 | H | C |
| 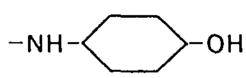 | 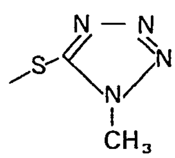 | H | D |
| 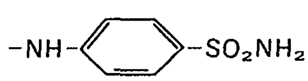 | $-OCOCH_3$ | H | E |
| 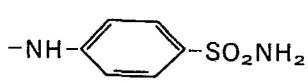 | 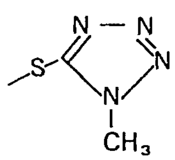 | H | F |
| 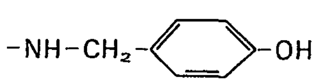 | 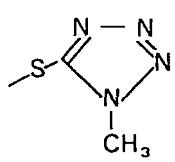 | H | G |

Als Vergleichsverbindungen wurden Cefuroxim und Cephazolin, zwei bekannte und gut wirksame Cephalosporine, herangezogen.

Minimale Hemmkonzentration (µg/ml)

| Substanz | *St. aureus* SG 511 | *E. coli* ATCC 11 775 | *Pseud. aeru.* Hbg. | *Pseud. aeru.* Walter | *Klebs. pneum.* ATCC 10 031 | *Klebs. pneum.* BC 6 | *Prot. mir.* BC 17 | *Prot. rettg.* BC 17 | *Enterob. cloacae* ATCC 13 047 | *E. coli* R+TEM | *Serr. marcesc.* ATCC 13 880 | *Klebs. pneum.* 1082 E |
|---|---|---|---|---|---|---|---|---|---|---|---|---|
| Cefuroxim | 1 | 8 | >128 | >128 | 2 | 4 | 0,5 | 2 | 32 | 4 | 8 | — |
| Cephazolin | 0,06 | 4 | >128 | >128 | 1 | 2 | 4 | >128 | >128 | 4 | >128 | >128 |
| A | 1 | 0,03 | 32 | 32 | 0,03 | 0,06 | 0,03 | 0,12 | 0,5 | 0,5 | 0,25 | 32 |
| B | 1 | 0,06 | 32 | 32 | 0,12 | 0,06 | 0,06 | 0,12 | 0,25 | 0,5 | 0,25 | 16 |
| C | 2 | 0,06 | 32 | 32 | 0,25 | 0,12 | 0,06 | 0,25 | 0,5 | 1 | 0,25 | 32 |
| D | 2 | 0,06 | 16 | 16 | 0,06 | 0,06 | 0,12 | 0,12 | 0,25 | 1 | 0,25 | 32 |
| E | 0,5 | 0,03 | 16 | 8 | 0,06 | 0,06 | 0,12 | 0,12 | 0,25 | 1 | 0,12 | 32 |
| F | 1 | 0,06 | 16 | 16 | 0,12 | 0,12 | 0,25 | 0,25 | 0,5 | 2 | 0,5 | 64 |
| G | 0,5 | 0,03 | 16 | 8 | 0,06 | 0,03 | 0,03 | 0,06 | 0,12 | 0,5 | 0,12 | 16 |

Wie aus der Tabelle ersichtlich sind die genannten Verbindungen den Vergleichssubstanzen in ihrer Wirksamkeit gegenüber typischen gramnegativen Hospitalkeimen bei Erhaltung der Wirkung gegen grampositive Keime deutlich überlegen.

Die akute Toxizität wurde durch perorale und subcutane Applikation der Verbindungen der beiden Tabelle an weissen Laboratoriumsmäusen in steigenden Dosen bestimmt.

Die $LD_{50}$ ist die Dosis, nach deren Applikation 50% der Tiere innerhalb von 8 d sterben. Sämtliche Substanzen zeigten bei oraler Gabe eine $LD_{50}$ von über 4 g/kg, bei subkutaner Gabe eine $LD_{50}$ von über 2 g/kg, d.h. bei 2 g/kg starben keine Tiere, sie sind damit für die Praxis untoxisch.

Eine Reihe der erfindungsgemässen Verbindungen wurde *in vivo* nach experimentellen Infektionen an Mäusen untersucht. Man verwendete als pathogene Bakterien *E. coli* ATCC 11 775. Es wurde eine intraperitoneale Infektion mit 0,2 ml einer Bakteriensuspension (mit 5% Mucin) gesetzt. Dies entspricht etwa $1{,}4 \times 10^6$ Keime *E. coli*/Maus. Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemässen Cephalosporine subkutan zur Bestimmung der $ED_{50}$ (Dosis bei der 50% der Tiere überleben) behandelt. Es wurde einmal 1 h nach der Infektion therapiert.

Der Beobachtungszeitraum betrug in beiden Fällen 7 d. Die Ergebnisse dieser Teste mit repräsentativen Vertretern der erfindungsgemässen Cephalosporine sind in der folgenden Tabelle dargestellt:

| Verbindung | $ED_{50}$ (µg/kg) |
|---|---|
| A | 0,5 |
| D | <0,3 |
| E | 0,1-0,3 |
| G | <0,2 |
| Cefuroxim | >100 |
| Cefaperazone (T 1551) | 3,1 |

Es ist eine weitere Aufgabe der Erfindung, pharmazeutische Mittel zu schaffen, die bei der Behandlung infektiöser Krankheiten sowohl beim Menschen als auch beim Tier wertvoll sind. Die Herstellung dieser pharmazeutischen Mittel erfolgt auf nicht chemischem Weg.

Als bevorzugte pharmazeutische Zubereitungen seien Tabletten, Dragées, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I in einer Dosierung zwischen 5 und 500, vorzugsweise 10 bis 200 mg/kg Körpergewicht je 24 h verabreicht, gegebenenfalls in Form mehrerer Einzelgaben. Eine Einzelgabe enthält den oder die erfindungsgemässen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch

kann eine Infektion durch gramnegative und grampositive Bakterien verhindert, gebessert und/oder geheilt werden und ebenso eine Förderung des Wachstums und eine Verbesserung der Verwertung des Futters erreicht werden.

Die folgenden Beispiele sollen die Erfindung näher erläutern:

*Beispiel 1:*

*Natrium-7β-{D,L-α-[(2-cyclopropylamino-4-hydroxy-5-pyrimidinyl)ureido]-(2.3-dihydro-2-imino-4-thiazolyl)acetamido}-3-[(1-methyl-tetrazol-5-yl)thiomethyl]ceph-3-em-4-carboxylat*

302 mg (2 mmol) 5-Amino-2-cyclopropyl-4-hydroxypyrimidin werden in einer kleinen Menge trockenem Tetrahydrofurans gelöst und mit 0,27 ml Triäthylamin versetzt. Diese Lösung wird bei 0°C zu einer Lösung von 200 mg Phosgen in 15 ml trockenem Tetrahydrofuran getropft. Man lässt 5 min bei Raumtemperatur rühren und engt dann im Vakuum auf die Hälfte des Volumens ein.

1,2 g (2 mmol) 7β-[D,L-α-Amino-(2.3-dihydro-2-imino-4-thiazolyl)acetamido]-3-[(1-methyltetrazol-5-yl)thiomethyl]ceph-3-em-4-carbonsäuretrifluoracetat (Synthese s. DE-OS Nr. 2924296, S. 35) werden unter Eiskühlung in einem Gemisch aus 30 ml Tetrahydrofuran und 20 ml Wasser vorgelegt. Der pH-Wert wird mit verdünnter Natronlauge vorsichtig auf 8,5 bis 9,0 eingestellt. Unter Eiskühlung wird das oben hergestellte Gemisch zugetropft, wobei der pH-Wert mit verdünnter Natronlauge auf etwa 8,0 gehalten wird. Nach der Zugabe wird 1 h bei 5°C und 1 h bei Raumtemperatur nachgerührt. Anschliessend wird mit etwas Wasser verdünnt und das Tetrahydrofuran im Wasserstrahlvakuum abgezogen. Die zurückbleibende wässerige Phase wird zweimal mit Essigster ausgeschüttelt. Die wässerige Phase wird dann an einem Ionenaustauscherharz Amberlite XAD-2 chromatographiert und zunächst mit Wasser und hierauf mit einem Gemisch von Wasser und Methanol im Verhältnis 75:25 eluiert. Die produkthaltigen Fraktionen werden gefriergetrocknet.

Ausbeute: 700 mg (53%)

Die freie Säure wird in Wasser suspendiert und durch Zusatz von Natriumhydrogencarbonat gelöst. Die erhaltene Lösung wird gefriergetrocknet. Man erhält ein weisses Pulver.

IR-Spektrum: 1765, 1660, 1610 $cm^{-1}$

NMR-Spektrum (DMSO + $CD_3OD$) Signale bei ppm: 1,25 (m, 4H), 1,90 (m, 1H), 3,50 (q, 2H), 3,90 (s, 3H), 4,20 (q, 2H; teilweise durch LM verdeckt), 4,95 (dd, 1H), 5,3 (d, 1H), 5,65 (dd, 1H), 6,55 (d, 1H), 8,55 (s, 1H)

*Beispiel 2:*

*Natrium-7β-{D,L-α-[(4-hydroxy-2-propyl-amino-5-pyrimidinyl)ureido]-(2,3-dihydro-2-imino-4-thiazolyl)acetamido}-3-[(1-methyl-tetrazol-5-yl)thiomethyl]ceph-3-em-4-carboxylat*

Dieses Cephalosporin wurde analog Beispiel 1 hergestellt. Man geht aus von 170 mg (1 mmol) 5-Amino-4-hydroxy-2-propylaminopyrimidin, das nach Umsetzung mit 0,13 ml Triäthylamin und 100 mg Phosgen in Tetrahydrofuran analog Beispiel 1 mit 600 mg (1 mmol) des dort eingesetzten Cephalosporinderivats umgesetzt wird.

Die Aufarbeitung erfolgt analog Beispiel 1.

Ausbeute: 305 mg Säure (46%)

NMR-Spektrum (DMSO + $CD_3OD$) Signale bei ppm: 1,0 (t, 3H), 1,6 (m, 2H), 3,2 (t, 2H), 3,65 (q, 2H), 3,90 (s, 3H), 4,25 (q, 2H), 5,15 (dd, 1H), 5,40 (d, 1H), 5,65 (dd, 1H), 6,60 (s, 1H), 8,10 (s, 1H)

*Beispiel 3:*

*Natrium-7β-{D,L-α-[(4-hydroxy-2-(3′-hydroxypropylamino)-5-pyrimidinyl)ureido]-(2,3-dihydro-2-imino-4-thiazolyl)acetamido}-3-[(1-methyltetrazol-5-yl)thiomethyl]ceph-3-em-4-carboxylat*

500 mg (2,7 mmol) 5-Amino-4-hydroxy-2-(3′-hydroxypropylamino)pyrimidin werden 5 min lang mit 3 ml Trimethylsilyldiäthylamin auf 80°C erwärmt. Das homogene Gemisch wird im Vakuum zur Trockne eingeengt und das erhaltene Festprodukt in 30 ml trockenem Tetrahydrofuran gelöst. Diese Lösung wird unter Eiskühlung zu einer Lösung von 275 mg Phosgen in 20 ml Tetrahydrofuran getropft.

Die weitere Umsetzung erfolgt analog Beispiel 1.

Ausbeute: 1,14 g (61%)

IR-Spektrum: 1765, 1650, 1610 $cm^{-1}$

NMR-Spektrum (DMSO + $CD_3OD$) Signale bei ppm: 1,85 (m, 2H), 3,3 (m, 2H), 3,45 (q, 2H), 3,6 (m, 2H), 3,95 (s, 3H), 4,30 (q, 2H), 4,95 (dd, 1H), 5,3 (s, 1H), 5,55 (dd, 1H), 6,40 (d, 1H), 8,15 (s, 1H)

*Beispiel 4:*

*Natrium-7β-{D,L-α-[(2-p-aminosulfonylanilino-4-hydroxy-5-pyrimidinyl)-ureido]-(2.3-dihydro-2-imino-4-thiazolyl)-acetamido-}-3-acetoxymethyl-ceph-3-em-4-carboxylat*

7β-[D,L-α-Amino-(2.3-dihydro-2-(chloracetyl)imino-4-thiazolyl)acetamido]-3-acetoxymethylceph-3-em-4-carbonsäuretrifluoracetat wird entsprechend der DE-OS Nr. 2924296, S. 24 f, synthetisiert.

1,4 g (5 mmol) 5-Amino-2-p-aminosulfonylanilino-4-hydroxypyrimidin werden entsprechend der in Beispiel 3 angegebenen Verfahrensweise silyliert und mit Phosgen umgesetzt. Die Umsetzung mit dem Cephalosporinderivat erfolgt entsprechend Beispiel 1, wobei wie folgt aufgearbeitet wird: Die wässerige Phase wird bei einem pH-Wert von 8,0 zweimal mit Essigester ausgeschüttelt und dann unter Eiskühlung mit 2N-Salzsäure auf einem pH-Wert von 3,0 gestellt. Das ausgefallene Produkt wird abgesaugt und getrocknet. Es werden 2,76 g (66%) rohes (an der Iminogruppe durch Chloracetyl geschütztes) Cephalosporinderivat erhalten.

Man löst diese freie Säure in Methanol und gibt eine Lösung von Diphenyldiazomethan in Dioxan zu. Man rührt 3 h bei Raumtemperatur und saugt

dann den entstandenen Niederschlag ab, wobei man 2,42 g Diphenylmethylester erhält (74%).

1 g des so erhaltenen Diphenylmethylesters und 200 mg Thioharnstoff werden in 100 ml eines Gemisches aus Chloroform, Methanol und Dioxan (2:1:1) 2 h zum Rückfluss erhitzt. Die erhaltene Lösung wird im Vakuum zur Trockne eingeengt und der Rückstand mit Wasser versetzt. Man erhält 800 mg des Diphenylmethylesters der Titelverbindung.

Diese 800 mg werden in einem Gemisch von 5 ml Methylenchlorid, 8 ml Trifluoressigsäure und 4 ml Anisol 30 min unter Eiskühlung gerührt. Anschliessend wird im Vakuum zur Trockne eingeengt und der Rückstand mit Äther digeriert. Das Festprodukt wird abgesaugt und mit Natriumhydrogencarbonat in das Natriumsalz überführt.

IR-Spektrum: 1765, 1660, 1610 $cm^{-1}$

NMR-Spektrum (DMSO + $CD_3OD$) Signale bei ppm: 2,05 (s, 3H), 3,45 (q, 2H), 4,75 (q, 2H), 4,90 (dd, 1H), 5,35 (s, 1H), 5,65 (dd, 1H), 6,55 (d, 1H), 7,83 (dd, 4H), 8,32 (s, 1H)

*Beispiel 5:*

*Natrium-7β-{D,L-α-[(2-p-aminosulfonylanilino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat*

Diese Verbindung wird analog Beispiel 4 synthetisiert. Man geht aus von 1,0 g (3,57 mmol) des dort angegebenen Pyrimidins sowie 2,0 g (3,6 mmol) 7-[D,L-α-Amino-(2,3-dihydro-2-(chloracetyl)imino-4-thiazolyl)acetamido]-3-[(1-methyltetrazol-5-yl)thiomethyl]ceph-3-em-4-carbonsäure.

Ausbeute (nach Überführung in den Diphenylmethylester, Abspaltung der Chloracetylgruppe und der Estergruppe) an Säure: 1,09 g (38,5%).

IR-Spektrum: 1765, 1655, 1605 $cm^{-1}$ (Natriumsalz)

NMR-Spektrum (DMSO + $CD_3OD$) Signale bei ppm: 3,45 (q, 2H), 3,95 (s, 3H), 4,25 (q, 2H), 4,90 (dd, 1H), 5,30 (d, 1H), 5,65 (dd, 1H), 6,55 (d, 1H), 7,80 (dd, 4H), 8,33 (s, 1H)

*Beispiel 6:*

*Natrium-7β-{D,L-α-[(4-hydroxy-2-p-methyl-aminosulfonylanilino-5-pyrimidinyl)ureido]-(2,3-dihydro-2-imino-4-thiazolyl)acetamido}-3-[(1-methyltetrazol-5-yl)thiomethyl]ceph-3-em-4-carboxylat*

Dieses Cephalosporin wird analog Beispiel 1 hergestellt. Man geht aus von 590 mg (2 mmol) 5-Amino-4-hydroxy-2-p-methylaminosulfonyl-anilinopyrimidin, das nach Silylierung und Reaktion mit Phosgen mit 1,2 g des Cephalosporinderivats des Beispiels 1 umgesetzt wurde.

Ausbeute: 680 mg Natriumsalz (41%)

IS-Spektrum: 1765, 1655, 1615 $cm^{-1}$

NMR-Spektrum (DMSO + $CD_3OD$) Signale bei ppm: 2,35 (s, 3H), 3,45 (q, 2H), 3,90 (s, 3H), 4,30 (q, 2H), 4,90 (dd, 1H), 5,30 (d, 1H), 5,70 (dd, 1H), 6,60 (d, 1H), 7,8 (dd, 4H), 8,30 (s, 1H)

*Beispiel 7:*

a) *D,L-α-3-(4-Hydroxy-2-(4'-hydroxycyclo-hexylamino)-5-pyrimidinyl)ureido]-(2,3-dihydro-2-imino-4-thiazolyl)essigsäure*

1,73 g D,L-α-Amino-(2,3-dihydro-2-imino-4-thiazolyl)essigsäure (0,01 mol) werden mit 10 ml 1N-Natronlauge in einem Gemisch aus 60 ml Tetrahydrofuran und 20 ml Wasser gelöst.

2,24 g 5-Amino-4-hydroxy-2-(4'-hydroxycyclohexylamino)pyrimidin (0,01 mol) werden in 50 ml trockenen Tetrahydrofuran aufgeschlämmt und mit 6 ml Diäthylaminotrimethylsilan bis zur Lösung zum Rückfluss erhitzt. Man engt im Vakuum zur Trockne ein, löst wieder in 50 ml Tetrahydrofuran und tropft diese Lösung unter Eiskühlung zu einer Lösung von 1,05 g Phosgen in 20 ml trockenem Tetrahydrofuran. Man rührt 15 min bei Eiskühlung nach und engt dann im Vakuum auf das halbe Volumen ein.

Diese Lösung wird bei Eiskühlung zu der oben hergestellten Lösung zugetropft; dabei wird der pH-Wert auf 8,0 gehalten. Man entfernt die Kühlung und rührt 1 h bei Raumtemperatur nach. Anschliessend wird mit 30 ml Wasser verdünnt und das Tetrahydrofuran im Vakuum entfernt. Die wässerige Phase wird zweimal mit Essigester ausgeschüttelt, hierauf wird die wässerige Phase mit 2N-Salzsäure auf einem-pH-Wert von 3,8 gestellt. Der ausgefallene Niederschlag wird abgesaugt und im Vakuum getrocknet.

Ausbeute: 2,35 g (56%)

IR-Spektrum: 3300 (breit), 1640-1650, 1530 (breites Signal), 1155 $cm^{-1}$

NMR-Spektrum: (DMSO + $CD_3OD$) Signale bei ppm: 1,35 (m, 4H), 1,9 (m, 4H), 3,55 (m, 2H), 5,15 (s, 1H), 6,5 (s, 1H), 8,1 (s, 1H)

b) *Natrium-7β-{D,L-α-[3-(4-hydroxy-2-(4'-hydroxycyclohexylamino)-5-pyrimidinyl)-ureido]-(2,3-dihydro-2-imino-4-thiazolyl)-acetamido}-3-[(1-methyltetrazol-5-yl)thiomethyl]ceph-3-em-4-carboxylat*

2,08 g (0,05 mol) der Ureidocarbonsäure des Beispiels 7a werden in 50 ml trockenem Dimethylformamid gelöst. Man gibt 2,5 g 7-Amino-3-[(1-methyltetrazol-5-yl)thiomethyl]ceph-3-em-4-carboxylat, gelöst in 30 ml Methylenchlorid, zu und versetzt unter Eiskühlung mit 1,15 g Dicyclohexylcarbodiimid.

Man rührt über Nacht unter Eiskühlung, engt dann im Vakuum zur Trockene ein und rührt den Rückstand mit 50 ml Methanol und dann mit 100 ml Methylenchlorid aus. Das abgesaugte Festprodukt wird zur Enfernung geringfügiger Verunreinigungen über eine Silicagelsäule chromatographiert (Eluens Methylenchlorid/Methanol 5:1).

Der erhaltene Diphenylmethylester wird mit 10 ml Trifluoressigsäure und 4 ml Anisol in üblicher Weise gespalten und der Rückstand mit Natriumäthylhexanoat in Dimethylformamid/Methanol in das Natriumsalz überführt.

Ausbeute: 1,90 g (31%)

IR-Spektrum: 3340, 1765, 1660, 1540 $cm^{-1}$

NMR-Spektrum (DMSO + $CD_3OD$) Signale

bei ppm: 1,3 (m, 4H), 1,9 (m, 4H), 3,30-3,80 (m, 4H), 3,95 (s, 3H), 4,3 (m, 2H), 5,05 (m, 1H), 5,4 (s, 1H), 5,65 (dd, 1H), 6,50 (d, 1H), 8,1 (s, 1H)

Nach dieser Methode wurden die folgenden Cephalosporine der allgemeinen Formel I synthetisiert (E = Natriumion, Y = Wasserstoff):

| Beispiel | R | D | IR-Spektrum ($cm^{-1}$) | NMR-Spektrum (DMSO + $CD_3OD$) Signale bei ppm |
|---|---|---|---|---|
| 8 | 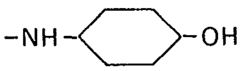 | $-OCOCH_3$ | 1765<br>1660 | 1,35 (m, 4H), 1,9 (m, 4H), 2,05 (s, 3H), 3,5 (m, 4H), 4,9 (m, 2H), 5,05 (d, 1H), 5,4 (s, 1H), 5,65 (q, 1H), 6,50 (d, 1H), 8,15 (s, 1H) |
| 9 | $-NH(CH_2)_3OH$ | $-OCOCH_3$ | 1760<br>1650 | 1,85 (m, 2H), 2,05 (s, 3H), 3,3 (m, 2H), 3,5-3,7 (m, 4H), 4,85 (m, 2H), 5,0 (dd, 1H), 5,35 (s, 1H), 5,60 (dd, 1H), 6,40 (s, 1H, breit), 8,15 (s, 1H) |
| 10 | $-NH(CH_2)_3CONH_2$ | 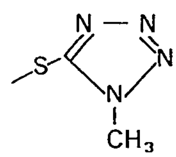 | 1765<br>1660 | 1,80 (m, 2H), 2,3 (m, 2H), 3,2-3,6 (m, 4H), 3,95 (s, 3H), 4,3 (q, 2H), 5,0 (d, 1H), 5,4 (s, 1H), 5,65 (dd, 1H), 6,50 (d, 1H), 8,10 (s, 1H) |
| 11 | 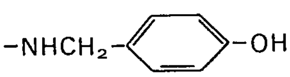 | 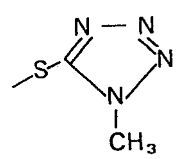 | 1760<br>1650<br>1610 | 3,55 (m, 2H), 3,95 (s, 3H), 4,35 (m, 4H), 4,95 (t, 1H), 5,30 (s, 1H), 5,55 (dd, 1H), 6,40 (d, 1H), 6,7 (d, 2H), 7,15 (d, 2H), 8,05 (s, 1H) |
| 12 | 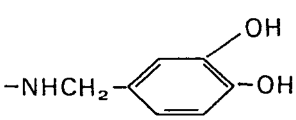 | 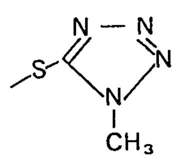 | 1760<br>1650 | 3,60 (m, 2H), 3,95 (s, 3H), 4,25 (m, 2H), 4,40 (s, breit, 2H), 4,95 (dd, 1H), 5,35 (s, 1H), 5,55 (dd, 1H), 6,40 (d, 1H), 6,7 (m, 2H), 7,1 (d, 1H), 8,05 (s, 1H) |
| 13 | 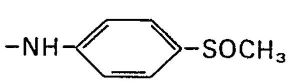 | 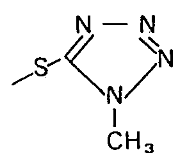 | 1765<br>1655<br>1605 | 2,80 (s, 3H), 3,55 (m, 2H), 3,90 (s, 3H), 4,3 (m, 2H), 5,0 (dd, 1H), 5,40 (s, 1H), 5,60 (dd, 1H), 6,45 (d, 1H), 7,60 (q, 4H), 8,33 (s, 1H) |
| 14 | 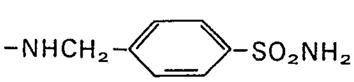 | 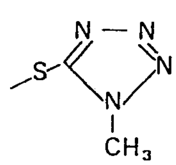 | 1725<br>1655 | (Säure)<br>3,70 (m, 2H), 3,95 (s, 3H), 4,3 (m, 2H), 4,65 (s, 2H), 5,10 (m, 1H), 5,40 (s, 1H), 5,70 (dd, 1H), 6,6 (d, 1H), 7,55 (d, 2H), 7,85 (d, 2H), 8,10 (s, 1H) |

*Beispiel 15:*

*Natrium-7β-{D,L-α-[(2-p-aminosulfonylanilino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat*

a) *D,L-α-Amino[2,3-dihydro-2-(chloracetyl)-imino-4-thiazolyl]essigsäuretrifluoracetat*

12,4 g (0,03 mol) D,L-α-[(4-Methoxyphenyl)-methoxycarbonyl]amino-[2,3-dihydro-2-(chloracetyl)imino-4-thiazolyl]essigsäure (Herstellung s. DE-OS Nr. 2924296) werden in 20 ml Methylenchlorid unter Eiskühlung 15 min lang mit 20 ml Trifluoressigsäure und 10 ml Anisol gerührt. Anschliessend wird im Vakuum zur Trockne eingeengt und der Rückstand mit Äther digeriert.

b) *D,L-α-(2-Aminosulfonylanilino-4-hydroxy-5-pyrimidinyl)ureido-(2.3-dihydro-2-(chloracetyl)imino-4-thiazol)essigsäure*

1,8 g (0,005 mol) der Verbindung der Stufe a werden mit 1N-Natronlauge in einem Gemisch von 50 ml Tetrahydrofuran und 20 ml Wasser gelöst. Man tropft unter Eiskühlung das Umsetzungsprodukt von 1,4 g (0,005 mol) 5-Amino-2-p-aminosulfonylanilino-4-hydroxypyrimidin (mit Trimethylsilyldiäthylamin und Phosgen), gelöst in trockenem Tetrahydrofuran zu. Dabei wird der pH-Wert auf 7,5 gehalten. Man lässt eine Stunde unter Eiskühlung und eine Stunde bei Raumtemperatur nachrühren. Dann verdünnt man mit etwas Wasser und zieht das Tetrahydrofuran im Vakuum ab. Die wässerige Phase wird zweimal mit Essigester ausgeschüttelt und dann auf einem pH-Wert von 3,0 angesäuert. Das ausgefallene Produkt wird abgesaugt und getrocknet.

Ausbeute: 1,87 g (67,5%).

c) 1,1 g der so erhaltenen Ureidocarbonsäure (2 mmol) werden in 25 ml Methylenchlorid bei −20°C mit 210 mg N-Methylmorpholin und 225 mg Chlorameisensäureäthylester versetzt. Man rührt 10 min bei −15°C und gibt dann eine Lösung von 1,00 g 7-β-Amino-3-[(1-methyl-tetrazol-5-yl)thiomethyl]ceph-3-em-4-carbonsäurediphenylmethylester in trockenem Methylenchlorid zu. Man rührt 1,5 h bei −10°C und 1 h bei Raumtemperatur. Anschliessend wird zur Trockne eingeengt. Der Rückstand wird einer Säulenchromatographie an Silicagel unterworfen (Eluens Methylenchlorid/Methanol 6:1).

d) 150 mg des so erhaltenen Diphenylmethylesters werden in 20 ml Tetrahydrofuran gelöst und mit einer Lösung von 20 mg Natrium-N-methyldithiocarbamat in 3 ml Wasser 5 h lang bei Raumtemperatur gerührt. Man engt im Vakuum zur Trockne ein, rührt den Rückstand mit 20 ml Methanol gut aus, saugt ab und wäscht mit Äther nach. Das so erhaltene Produkt wird auf übliche Weise mit Trifluoressigsäure/Anisol gespalten und in das Natriumsalz überführt; NMR- und IR-Spektren sind identisch mit denen der Verbindung des Beispiels 6.

Die so erhaltene D,L-Verbindung wird durch präparative Flüssigkeitschromatographie (HPLC) unter Verwendung einer 7 µm Umkehrphase-C8-Säule (Lichrosorb RP 8, Firma Merck, Darmstadt) in die D- und L-Form aufgetrennt. Als Eluat wird ein Gemisch von 1000 Teilen Wasser, 50 Teilen Methanol und 3 Teilen Natriumhydrogencarbonat verwendet. Das Eluat wird bei 254 nm (UV-Strahlung) verfolgt.

Auf analoge Weise wurden folgende Cephalosporine synthetisiert:

| Beispiel | R | D | IR-Spektrum ($cm^{-1}$) | NMR-Spektrum (DMSO + $CD_3OD$) Signale bei ppm |
|---|---|---|---|---|
| 16 | 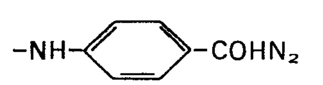 | 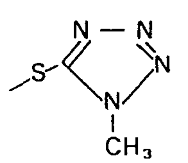 | 1760<br>1645<br>1605 | 3,60 (m, 2H), 3,95 (s, 3H), 4,3 (m, 2H), 5,0 (m, 1H), 5,45 (s, 1H), 5,65 (dd, 1H), 6,50 (d, 1H), 7,7 (q, 4H), 8,30 (s, 1H) |
| 17 | 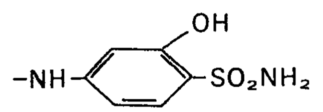 | 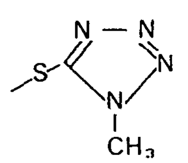 | 1765<br>1650 | 3,55 (m, 2H), 3,95 (s, 3H), 4,25 (m, 2H), 5,0 (m, 1H), 5,40 (s, 1H), 5,60 (dd, 1H), 6,45 (d, 1H), 7,35 (m, 1H), 7,75 (m, 2H), 8,34 (s, 1H) |

*Beispiel 18:*

*Natrium-7α-methoxy-7β-{D,L-α-[3-(4-hydroxy-2(4'-hydroxybenzylamino-5-pyrimidinyl)-ureido]-2,3-dihydro-2-imino-4-thiazolyl)-acetamido}-3-[(1-methyltetrazol-5-yl)thiomethyl]ceph-3-em-4-carboxylat*

1,8 g (0,002 mol) des Diphenylmethylesters des Cephalosporins des Beispiels 11 werden in 70 ml trockenem Tetrahydrofuran gelöst. Man gibt bei −70°C eine Lösung von 500 mg Lithiummethoxid in 20 ml trockenem Methanol zu und rührt bei dieser Temperatur 3 min. Dann fügt man bei −70°C 300 mg t-Butylhypochlorit zu. Man rührt 45 min bei −70°C, gibt dann 0,6 ml Eisessig und 150 mg Triäthylphosphit zu. Bei Raumtemperatur wird mit 100 ml Phosphatpuffer (pH-Wert 7,0) versetzt

und das Gemisch dreimal mit Methylenchlorid, dem etwas Tetrahydrofuran zugesetzt wird, extrahiert. Die organische Phase wird abgetrennt, getrocknet und das Lösungsmittel im Vakuum entfernt. Der Rückstand wird zweimal über eine Silicagelsäule chromatographiert (Eluens Methanol/Methylenchlorid 1:6). Man erhält 260 mg des gewünschten Diphenylmethylesters (14% der Theorie).

Die Spaltung zur Säure und die Überführung in das Natriumsalz werden wie im Beispiel 1 durchgeführt.

IR-Spektrum: 1765, 1670, 1155 $cm^{-1}$

NMR-Spektrum: (DMSO + $CH_3OD$) Signale bei ppm: 3,45 (s, 3H), 3,55 (m, 2H), 3,95 (s, 3H), 4,3 (m, 2H), 4,55 (s, 2H), 5,0 (s, 1H), 5,45 (s, 1H), 6,50 (d, 1H), 6,7 (d, 2H), 7,15 (d, 2H), 8,05 (s, 1H)

*Beispiel 19:*

*Natrium-7α-methoxy-7β-{D,L-α-[3-(4-hydroxy-2-(4'hydroxycyclohexylamino)-5-pyrimidinyl)ureido]-(2,3-dihydro-2-amino-4-thiazolyl)acetamido}-2-[(1-methyltetrazol-5-yl)thiomethyl]ceph-3-em-4-carboxylat*

In einem Gemisch aus 10 ml Methanol und 10 ml Methylenchlorid wurden 520 mg 7β-DL-[2-Amino-2-(2-aminothiazol-4-yl)acetamido]-α-methoxy-3-(1-methyltetrazol-5-yl)thiomethyl-cephem-4-carbonsäure (Synthese s. DE-OS Nr. 2927683) suspendiert. Dann wurden 0,30 ml Triäthylamin zu der Suspension hinzugefügt. Zu der erhaltenen Lösung wurde bei 0° C das entsprechend Beispiel 7a aus dem Aminopyrimidin (230 mg), Trimethylsilyldiäthylamin und Phosgen hergestellte Zwischenprodukt, suspendiert in Tetrahydrofuran, hinzugefügt. Das Gemisch wurde 5 h bei Raumtemperatur gerührt. Das Lösemittel wurde im Vakuum entfernt. Man fügte 20 ml Wasser und 30 ml Essigester zu dem Rückstand, stelle auf einem pH-Wert von 7,0 und trennte die Essigesterschicht ab. Dann wurde die wässerige Schicht mit 2N-Salzsäure bis zu einem pH-Wert von 3,6 angesäuert. Das ausgefallene Produkt wurde abgesaugt und wie üblich in das Natriumsalz überführt.

Ausbeute: 330 mg

IR-Spektrum: 1765, 1660, 1610 $cm^{-1}$

NMR-Spektrum (DMSO + $CH_3OD$) Signale bei ppm: 1,4 (m, 4H), 1,8 (m, 4H), 3,3-3,6 (m, 4H), 3,45 (s, 3H), 3,95 (s, 3H), 4,3 (m, 2H), 5,05 (s, 1H), 5,40 (s, 1H), 6,40 (d, 1H), 8,10 (s, 1H)

Analog wurden folgende 7α-Methoxycephalosporine hergestellt:

| Beispiel | R | D | IR-Spektrum ($cm^{-1}$) | NMR-Spektrum (DMSO + $CD_3OD$) Signale bei ppm |
|---|---|---|---|---|
| 20 | Cyclopropyl | 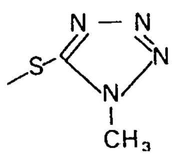 | 1760<br>1650 | 1,25 (m, 4H), 1,90 (m, 1H), 3,45 (m, 2H + s, 3H), 3,95 (s, 3H), 4,95 (s, 1H), 5,35 (s, 1H), 6,40 (d, 1H), 8,50 (s, 1H) |
| 21 | 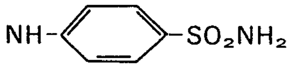 | 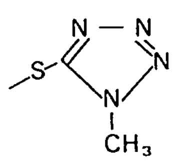 | 1765<br>1660 | 3,45 (s, 3H), 3,60 (m, 2H), 3,95 (s, 3H), 4,35 (m, 2H), 5,0 (s, 1H), 5,45 (s, 1H), 6,50 (d, 1H), 7,7 (dd, 4H), 8,32 (s, 1H) |
| 22 | 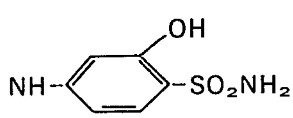 | 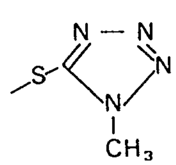 | 1765<br>1660 | 3,40 (s, 3H), 3,60 (m, 2H), 3,95 (s, 3H), 4,35 (m, 2H), 5,0 (s, 1H), 5,40 (s, 1H), 6,45 (d, 1H), 7,40 (m, 1H), 7,80 (m, 2H), 8,33 (s, 1H) |

*Beispiel 23:*

*Natrium-7β-{D,L-α-[3,(2-(p-aminosulfonylanilino)-4-hydroxy-5-pyrimidinyl)-ureido]-(2.3-dihydro-2-imino-4-thiazolyl)-acetamido}-3-[(2-methylthiadiazol-5-yl)-thiomethyl]ceph-3-em-4-carboxylat*

600 mg des Cephalosporins des Beispiels 4 werden zusammen mit 180 mg 2-Methyl-5-mercaptothiadiazol in 40 ml Nitromethan 6 h lang auf 80° C erwärmt. Man engt im Vakuum zur Trockne ein. Der Rückstand wird in einem Gemisch aus Aceton und Essigester gelöst. Unter Eiskühlung gibt man so lange Diphenyldiazomethan zu, bis die Violettfärbung erhalten bleibt. Man engt danach zur Trockne ein. Der Rückstand wird durch Säulenchromatographie gereinigt (Silicagel, Eluens Methylenchlorid/Methanol 5:1). Der erhaltene

Ester wird wie üblich gespalten und die Säure ins Natriumsalz überführt.

Ausbeute: 285 mg

IR-Spektrum: 1760, 1650, 1600 $cm^{-1}$

NMR-Spektrum (DMSO + $CD_3OD$) Signale bei ppm: 2,75 (s, 3H), 3,4 (m, 2H), 3,95 (s, 3H), 4,35 (m, 2H), 4,95 (t, 3H), 5,35 (s, breit, 1H), 5,60 (dd, 1H), 6,40 (d, 1H), 7,75 (q, 4H), 8,32 (s, 1H)

Beispiel zur Herstellung pharmazeutischer Darreichungsformen:

*Trockenampullen enthaltend Natrium-7β-{D,L-α-[(2-p-aminosulfonylanilino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carboxylat*

In einem aseptischen Bereich wurden 251 g Wirkstoff in 2008 ml destilliertem Wasser zur Injektion aufgelöst. Die Lösung wurde durch ein Millipore-Filter (Porengrösse 0,22 µm, Produkt der Millipore Corporation, Bedford, USA) filtriert. Die Lösung wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde.

Destilliertes Wasser zur Injektion wurde in einer Menge von 20 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

**Patentansprüche für die Vertragsstaaten:** BE, DE, FR, GB, IT, LU, NL, SE, CH

1. Cephalosporine der allgemeinen Formel I:

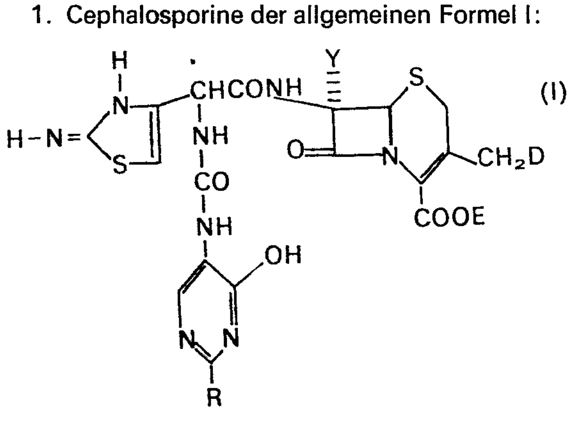

(I)

bzw. deren Tautomere der allgemeinen Formeln I', I'' und I''':

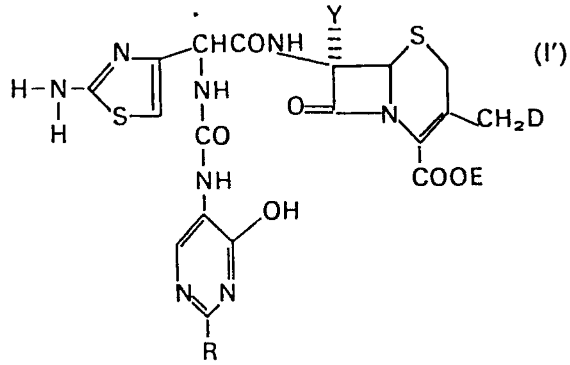

(I')

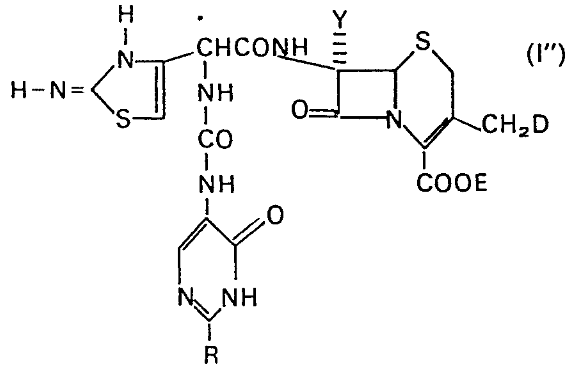

(I'')

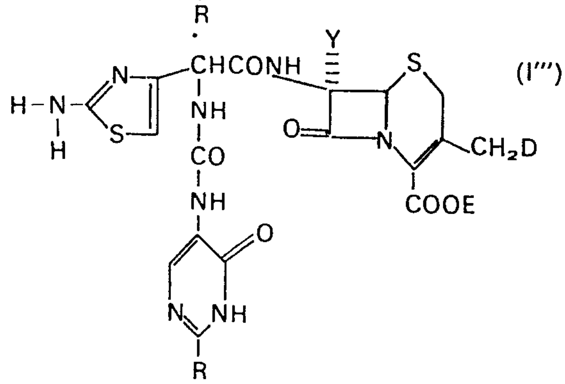

(I''')

worin

Y, das in der α-Konfiguration steht, ein Wasserstoffatom oder die Methoxygruppe,

D die Acetoxygruppe oder die Gruppe SHet, wobei Het die 1-Methyltetrazol-5-yl oder 2-Methyl-1,3,4-thiadiazol-5-ylgruppe bedeutet, und

E ein Wasserstoffatom oder ein Alkali- oder Erdalkalimetallion oder eine *in vitro* oder *in vivo* leicht abspaltbare Schutzgruppe darstellen, und

R die folgenden Bedeutungen besitzt: die Cyclopropylgruppe oder eine Gruppe der allgemeinen Formel $-NHR_1$, worin $R_1$ die Methyl-, Äthyl-, Propyl-, Isopropyl-, Cyclopentyl- oder Cyclohexylgruppe darstellt, oder R eine Gruppe der allgemeinen Formel $-NH-Z-X$, bei der $-NH-Z-$ eine Gruppe der Formeln

$$-\underset{H}{\underset{|}{N}}-CH_2-CH_2- \text{ oder } -\underset{H}{\underset{|}{N}}-(CH_2)_3-$$

darstellt, und

X die Hydroxy-, Methoxy-, Aminocarbonyl- oder Aminosulfonylgruppe bedeutet oder in der

$$-\underset{H}{\underset{|}{N}}-Z-X$$

die 4'-Hydroxycyclohexylaminogruppe bedeutet, R kann auch eine Gruppe der allgemeinen Formel:

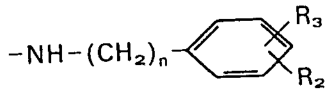

bedeuten, in der n=0 oder 1 ist und einer oder zwei der Reste $R_2$ und $R_3$ Wasserstoffatome, Chloratome, Methyl-, Hydroxy-, Acetylamino-, Methylsulfinyl-, Methylsulfonyl-, Aminocarbonyl-, Methylaminocarbonyl-, Aminosulfonyl-, Methylaminosulfonyl- oder Dimethylaminosulfonylgruppen oder Aminocarbonylaminogruppen darstellen, und ihre physiologisch verträgliche Salze mit anorganischen oder organischen Basen.

2. Cephalosporine der allgemeinen Formeln I, I', I'' und I''', gemäss Anspruch 1, in welchen Y, D und E wie im Anspruch 1 angegeben definiert sind und R eine p-Aminosulfonylanilino-, p-Methylsulfinylanilino-, p-Methylsulfonylanilino-, m-Hydroxy-p-aminosulfonylanilino-, m,p-Bis(aminocarbonyl)anilino, p-Aminosulfonylbenzylamino-, m,p-Dihydroxybenzylamino- oder p-Hydroxybenzylaminogruppe bedeutet.

3. Cephalosporine der allgemeinen Formeln I, I', I'' und I''' gemäss den Ansprüchen 1 und 2, dadurch gekennzeichnet, dass E als eine *in vitro* oder *in vivo* leicht abspaltbare Schutzgruppe die Benzyl-, Diphenylmethyl-, Trityl-, t-Butyl-, 2,2,2-Trichloräthyl- oder Trimethylsilylgruppe, eine Alkanoyloxyalkylgruppe mit 1 bis 5 Kohlenstoffatomen im Alkanoylrest und 1 bis 3 Kohlenstoffatomen im Alkylenrest oder Phthalidylgruppe bedeutet.

4. Cephalosporine der allgemeinen Formeln I, I', I'' und I''' gemäss Anspruch 3, dadurch gekennzeichnet, dass E die Acetoxymethyl-, Propionyloxymethyl-, 2-Acetoxyäthyl- oder Pivaloyloxymethylgruppe darstellt.

5. 7-{D,L-α-[(2-p-Aminosulfonylanilino-4-hydroxy-5-pyrimidinyl)ureido]-(2.3-dihydro-2-imino-4-thiazolyl)acetamido}-3-[(1-methyltetrazol-5-yl)thiomethyl]ceph-3-em-4-carbonsäure und ihre physiologisch verträgliche Salze mit anorganischen oder organischen Basen.

6. Verfahren zur Herstellung von Cephalosporinen der allgemeinen Formel I bzw. von deren Tautomeren der allgemeinen Formeln I', I'' und I''' gemäss Anspruch 1, worin D, E, Y und R wie im Anspruch 1 angegeben definiert sind, und, falls E ein Wasserstoffatom bedeutet, von deren Salzen mit anorganischen oder organischen Basen, dadurch gekennzeichnet, dass

a) ein 7-Aminocephalosporansäurederivat der allgemeinen Formel II:

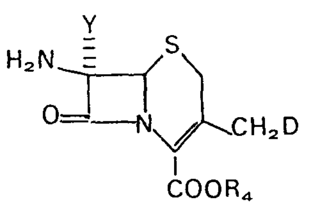

(II)

in der D wie oben definiert ist, und $R_4$ die tert.-Butyl-, Benzyl-, Diphenylmethyl-, Trimethylsilyl- oder 2,2,2-Trichloräthylgruppe darstellt, mit einer Ureidocarbonsäure der allgemeinen Formel III:

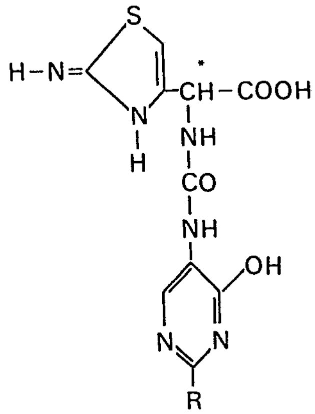

(III)

in der R die obigen Bedeutungen besitzt, oder mit ihren Salzen oder reaktiven Derivaten in einem Lösungsmittel bei Temperaturen zwischen −40 und +40°C zu einem Zwischenprodukt der allgemeinen Formel IV:

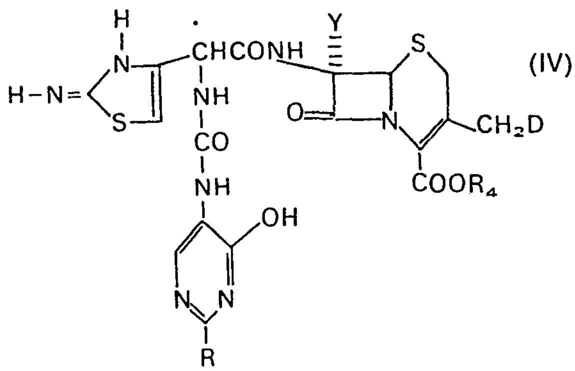

(IV)

umgesetzt und aus letzterem anschliessend der Rest $R_4$ hydrolytisch abgespalten wird, wobei ein Endprodukt der allgemeinen Formeln I, I', I'' und I''' entsteht, in welchem E ein Wasserstoffatom darstellt, oder

b) eine 7-Aminocephalosporansäure der allgemeinen Formel V:

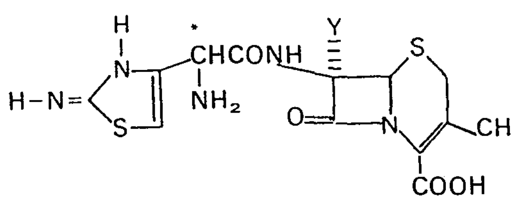

(V)

in der D und Y wie oben definiert sind oder ihre Salze mit einem Pyrimidinderivat der allgemeinen Formel VI oder VIa:

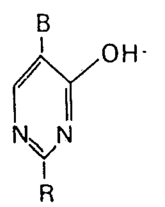

(VI)

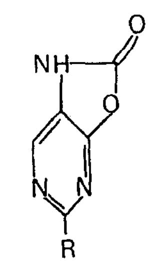

(VIa)

wobei in der allgemeinen Formel VI R wie oben definiert ist und B die Gruppe −NCO oder ein reaktives Derivat der Gruppe $-NHCOOH$, die Gruppe $-NHCOCl$, $-NHCOBr$ oder

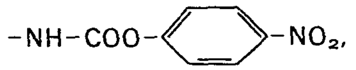

bedeutet, oder mit Gemischen solcher Pyrimidinderivaten, in welchen B teils die eine und teils die andere der vorstehend genannten Bedeutungen besitzt, in einem Lösungsmittel und bei einem pH-Bereich zwischen 2,0 und 9,0 bei Temperaturen zwischen −20 und +50°C umgesetzt wird, oder

c) zur Herstellung von Verbindungen der allgemeinen Formel I, I′, I″ bzw. I‴, in der D die Gruppe SHet mit den oben für Het angegebenen Bedeutungen bedeutet und E für Wasserstoff steht, eine Verbindung der allgemeinen Formel VII:

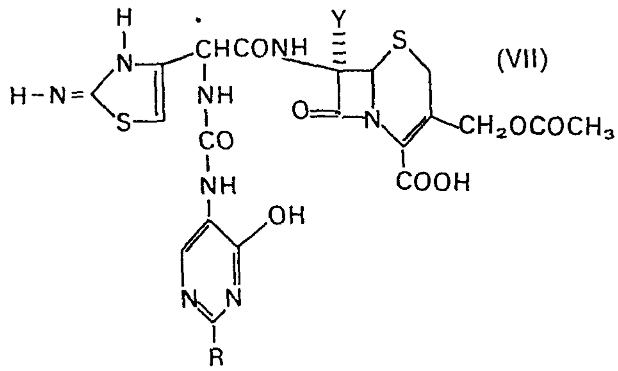

(VII)

in der R und Y die oben angegebenen Bedeutungen haben, mit einer Verbindung der allgemeinen Formel VIII

$$\text{Het-S-M} \qquad \text{(VIII)}$$

in der Het die oben angegebenen Bedeutungen hat und M für ein Wasserstoffatom oder ein Alkalimetall- oder ein Erdalkalimetallatom steht, in einem Lösungsmittel bei Temperaturen zwischen 0 und 100°C in einem pH-Bereich zwischen 2 und 10 zu einer Verbindung der allgemeinen Formel I, I′, I″ bzw. I‴, in der D die Bedeutungen einer Gruppe −SHet und E die eines Wasserstoffatoms besitzen, umgesetzt wird, oder

d) zur Herstellung einer Verbindung der allgemeinen Formel I, I′, I″ oder I‴, in der Y die Methoxygruppe darstellt, eine Verbindung der allgemeinen Formel I, I′, I″ oder I‴, in der Y ein Wasserstoffatom bedeutet, in Anwesenheit von Methanol mit einem Alkalimetallmethylat der allgemeinen Formel $M^+OCH_3^-$, worin $M^+$ ein Alkalimetallion bedeutet, und dann mit einem Halogenierungsmittel in einem inerten Lösungsmittel bei Temperaturen zwischen −120 und −10°C umgesetzt wird, und, gewünschtenfalls anschliessend, eine so erhaltene Verbindung der allgemeinen Formel I, I′, I″ oder I‴, worin E eine *in vitro* oder *in vivo* leicht abspaltbare Schutzgruppe darstellt, in die freie Carbonsäure der allgemeinen Formeln I, I′, I″ oder I‴, in der E ein Wasserstoffatom ist, übergeführt wird und/oder eine Verbindung der allgemeinen Formeln I, I′, I″ oder I‴, in der E ein Wasserstoffatom bedeutet, in ihre Ester oder, mittels anorganischen oder organischen Basen, in die entsprechenden Salze übergeführt wird.

7. Verfahren gemäss Anspruch 6a, dadurch gekennzeichnet, dass man als reaktive Derivate der Ureidocarbonsäuren der allgemeinen Formel III deren Säureanhydride, deren reaktive Ester, deren reaktive Amide, deren Säurehalogenide oder Säureazide und als 7-Aminocephalosporansäurederivate der allgemeinen Formel II solche Verbindungen verwendet, bei welchen E die eingangs erwähnten Bedeutungen mit Ausnahme der eines Wasserstoffatoms besitzt, und die Umsetzung in einem Lösungsmittel, gegebenenfalls in Anwesenheit einer Base und/oder eines Kondensationsmittels erfolgt.

8. Verfahren gemäss Anspruch 6b, dadurch gekennzeichnet, dass eine Verbindung der allgemeinen Formel V oder eines Salzes mit anorganischen oder organischen Basen mit einer Verbindung der allgemeinen Formel VI bzw. VIa:

a) in Wasser oder in mit Wasser mischbaren Lösungsmitteln in Gegenwart von Wasser in einem pH-Bereich von 6,5 bis 8,0, oder

b) in wasserfreien Lösungsmitteln, oder

c) in einem Gemenge aus Wasser und mit Wasser nicht mischbaren Lösungsmitteln in einem pH-Bereich zwischen 6,5 und 8,0

umgesetzt wird, oder dass eine Verbindung der allgemeinen Formel V, in der das Wasserstoffatom der Carboxylgruppe durch eine Silylgruppe oder eine andere leicht abspaltbare Schutzgruppe ersetzt ist, mit einer Verbindung der allgemeinen Formel VI bzw. VIa in einem wasser- und hydroxylgruppenfreien bzw. aprotischen Lösungsmittel, gegebenenfalls in Gegenwart einer Base, umgesetzt wird.

9. Arzneimittel enthaltend eine oder mehrere Verbindungen der allgemeinen Formeln I, I′, I″ und I‴ gemäss den Ansprüchen 1 bis 5 neben einem oder mehreren inerten Träger und/oder Hilfsstoffen.

10. Verwendung der Cephalosporine der allgemeinen Formeln I, I′, I″ und I‴ gemäss den Ansprüchen 1 bis 5 zur Herstellung von Arzneimitteln auf nicht chemischem Weg.

**Patentansprüche für den Vertragsstaat:** AT

1. Verfahren zur Herstellung von Cephalosporinen der allgemeinen Formel I:

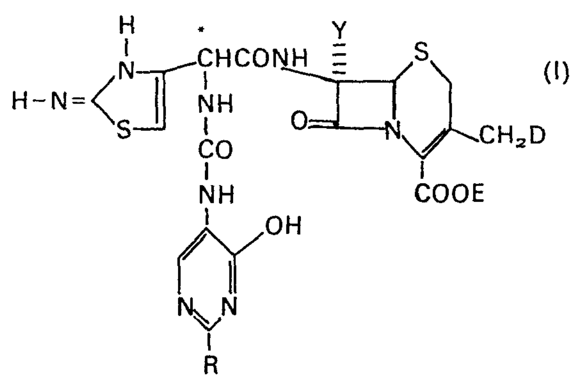

(I)

bzw. von deren Tautomeren der allgemeinen Formeln I′, I″ und I‴

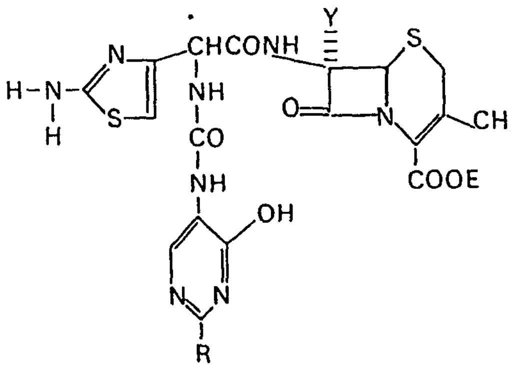

(I')

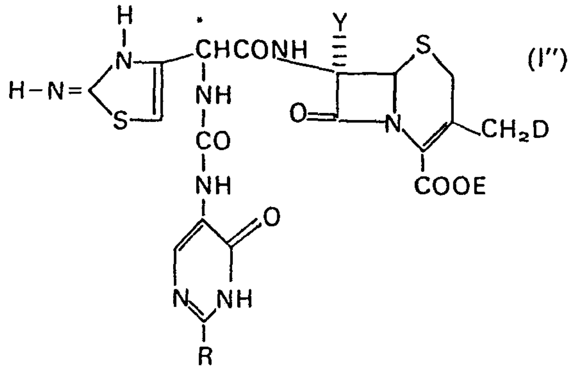

(I'')

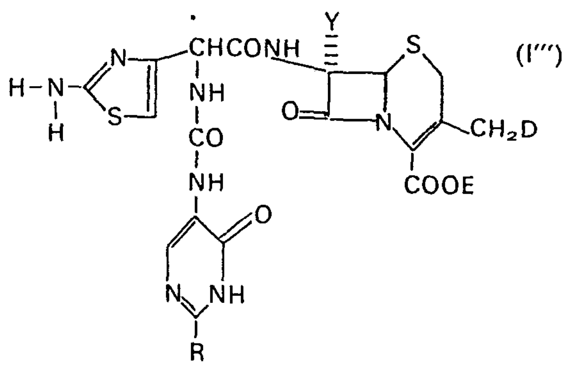

(I''')

worin

Y, das in der α-Konfiguration steht, ein Wasserstoffatom oder die Methoxygruppe,

D die Acetoxygruppe oder die Gruppe SHet, wobei Het die 1-Methyltetrazol-5-yl oder 2-Methyl-1,3,4-thiadiazol-5-ylgruppe darstellt,

E ein Wasserstoffatom oder ein Alkali- oder Erdalkalimetallion oder eine *in vitro* oder *in vivo* leicht abspaltbare Schutzgruppe bedeuten, und

R die folgenden Gruppen: die Cyclopropylgruppe oder eine Gruppe der allgemeinen Formel $-NHR_1$, worin $R_1$ die Methyl-, Äthyl-, Propyl-, Isopropyl-, Cyclopentyl- oder Cyclohexylgruppe darstellt, oder

R eine Gruppe der allgemeinen Formel $-NH-Z-X$, bei der $-NH-Z-$ eine Gruppe der Formeln $-NH-CH_2-CH_2-$ oder

$$-\underset{\displaystyle H}{N}-(CH_2)_3-$$

darstellt, und

X die Hydroxy-, Methoxy-, Aminocarbonyl- oder Aminosulfonylgruppe bedeutet oder in der $-NH-Z-X$ die 4'-Hydroxycyclohexylaminogruppe bedeutet,

R kann auch eine Gruppe der allgemeinen Formel:

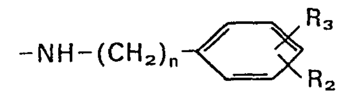

bedeuten, in der n=0 oder 1 ist und einer oder zwei der Reste $R_2$ und $R_3$ Wasserstoffatome, Chloratome, Methyl-, Hydroxy-, Acetylamino-, Methylsulfinyl-, Methylsulfonyl-, Aminocarbonyl-, Methylaminocarbonyl-, Aminosulfonyl-, Methylaminosulfonyl- oder Dimethylaminosulfonylgruppen oder Aminocarbonylaminogruppen darstellen, und von ihren physiologisch verträglichen Salzen mit anorganischen oder organischen Basen, dadurch gekennzeichnet, dass

a) ein 7-Aminocephalosporansäurederivat der allgemeinen Formel II:

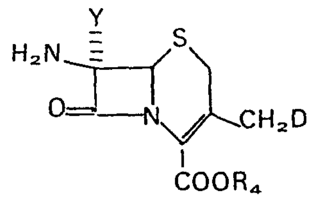

(II)

in der D wie oben definiert ist, und $R_4$ die tert.-Butyl-, Benzyl-, Diphenylmethyl-, Trimethylsilyl- oder 2,2,2-Trichloräthylgruppe darstellt, mit einer Ureidocarbonsäure der allgemeinen Formel III:

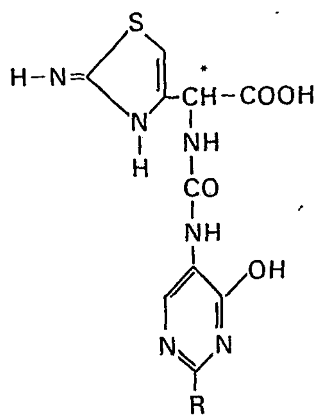

(III)

in der R die obigen Bedeutungen besitzt, oder mit ihren Salzen oder reaktiven Derivaten in einem Lösungsmittel bei Temperaturen zwischen −40 und +40°C zu einem Zwischenprodukt der allgemeinen Formel IV:

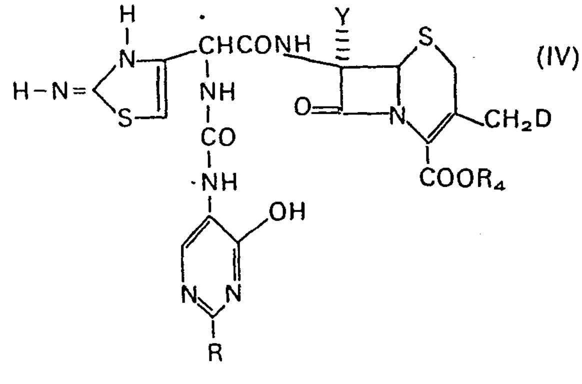

(IV)

umgesetzt und aus letzterem anschliessend der Rest $R_4$ hydrolytisch abgespalten wird, wobei ein

Endprodukt der allgemeinen Formeln I, I', I'' und I''' entsteht, in welchem E ein Wasserstoffatom darstellt, oder

b) eine 7-Aminocephalosporansäure der allgemeinen Formel V:

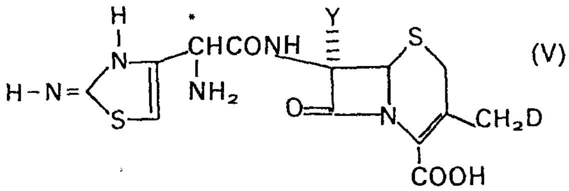

(V)

in der D und Y wie oben definiert sind oder ihre Salze mit einem Pyrimidinderivat der allgemeinen Formel VI oder VIa:

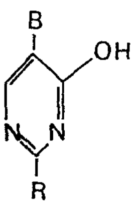

(VI)

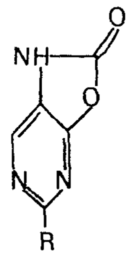

(VIa)

wobei in der allgemeinen Formel VI R wie oben definiert ist und B die Gruppe $-NCO$ oder ein reaktives Derivat der Gruppe $-NHCOOH$, die Gruppe $-NHCOCl$, $-NHCOBr$ oder

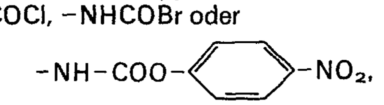

bedeutet, oder mit Gemischen solcher Pyrimidinderivaten, in welchen B teils die eine und teils die andere der vorstehend genannten Bedeutungen besitzt, in einem Lösungsmittel und bei einem pH-Bereich zwischen 2,0 und 9,0 bei Temperaturen zwischen −20 und +50°C umgesetzt wird, oder

c) zur Herstellung von Verbindungen der allgemeinen Formel I, I', I'' bzw. I''', in der D die Gruppe SHet mit den oben für Het angegebenen Bedeutungen bedeutet und E für Wasserstoff steht, eine Verbindung der allgemeinen Formel VII:

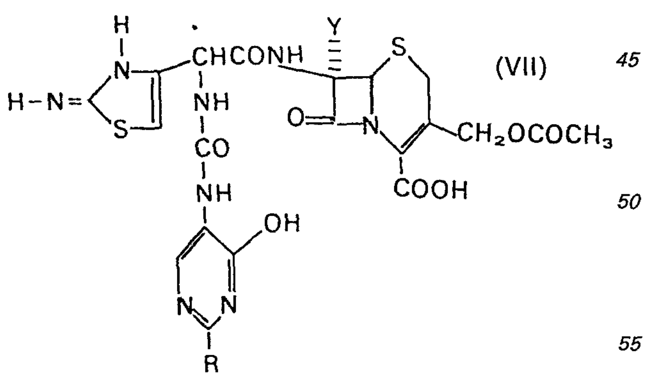

(VII)

in der R und Y die oben angegebenen Bedeutungen haben, mit einer Verbindung der allgemeinen Formel VIII:

$$Het-S-M \qquad (VIII)$$

in der Het die oben angegebenen Bedeutungen hat und M für ein Wasserstoffatom oder ein Alkalimetall- oder ein Erdalkalimetallatom steht, in einem Lösungsmittel bei Temperaturen zwischen 0 und 100°C in einem pH-Bereich zwischen 2 und 10 zu einer Verbindung der allgemeinen Formel I, I', I'' bzw. I''', in der D die Bedeutungen einer Gruppe −SHet und E die eines Wasserstoffatoms besitzen, umgesetzt wird, oder

d) zur Herstellung einer Verbindung der allgemeinen Formel I, I', I'' oder I''', in der Y die Methoxygruppe darstellt, eine Verbindung der allgemeinen Formel I, I', I'' oder I''', in der Y ein Wasserstoffatom bedeutet, in Anwesenheit von Methanol mit einem Alkalimetallmethylat der allgemeinen Formel $M^+OCH_3^-$, worin $M^+$ ein Alkalimetallion bedeutet, und dann mit einem Halogenierungsmittel in einem inerten Lösungsmittel bei Temperaturen zwischen −120 und −10°C umgesetzt wird, und, gewünschtenfalls anschliessend, eine so erhaltene Verbindung der allgemeinen Formel I, I', I'' oder I''', worin E eine *in vitro* oder *in vivo* leicht abspaltbare Schutzgruppe darstellt, in die freie Carbonsäure der allgemeinen Formeln I, I', I'' oder I''', in der E ein Wasserstoffatom ist, übergeführt wird und/oder eine Verbindung der allgemeinen Formeln I, I', I'' oder I''', in der E ein Wasserstoffatom bedeutet, in ihre Ester oder, mittels anorganischen oder organischen Basen, in die entsprechenden Salze übergeführt wird.

2. Verfahren gemäss Anspruch 1a, dadurch gekennzeichnet, dass man als reaktive Derivate der Ureidocarbonsäuren der allgemeinen Formel III deren Säureanhydride, deren reaktive Ester, deren reaktive Amide, deren Säurehalogenide oder Säureazide und als 7-Aminocephalosporansäurederivate der allgemeinen Formel II solche Verbindungen verwendet, bei welchen E die eingangs erwähnten Bedeutungen mit Ausnahme der eines Wasserstoffatoms besitzt, und die Umsetzung in einem Lösungsmittel, gegebenenfalls in Anwesenheit einer Base und/oder eines Kondensationsmittels erfolgt.

3. Verfahren gemäss Anspruch 1b, dadurch gekennzeichnet, dass eine Verbindung der allgemeinen Formel V oder eines ihrer Salze mit anorganischen oder organischen Basen mit einer Verbindung der allgemeinen Formel VI bzw. VIa:

a) in Wasser oder in mit Wasser mischbaren Lösungsmitteln in Gegenwart von Wasser in einem pH-Bereich von 6,5 bis 8,0, oder

b) in wasserfreien Lösungsmitteln, oder

c) in einem Gemenge aus Wasser und mit Wasser nicht mischbaren Lösungsmitteln in einem pH-Bereich zwischen 6,5 und 8,0

umgesetzt wird, oder dass eine Verbindung der allgemeinen Formel V, in der das Wasserstoffatom der Carboxylgruppe durch eine Silylgruppe oder eine andere leicht abspaltbare Schutzgruppe ersetzt ist, mit einer Verbindung der allgemeinen Formel VI oder VIa, in einem wasser- und hydroxylgruppenfreien bzw. aprotischen Lösungsmittel, gegebenenfalls in Gegenwart einer Base, umgesetzt wird.

4. Verfahren gemäss Anspruch 1c, dadurch gekennzeichnet, dass die Umsetzung in einem stark polaren Lösungsmittel bei einem pH-Wert von 4 bis 8 durchgeführt wird.

5. Verfahren gemäss Anspruch 1d, dadurch ge-

kennzeichnet, dass eine Verbindung der allgemeinen Formel I, I', I'' oder I''', worin Y ein Wasserstoffatom bedeutet, in einem inerten Mittel gelöst oder suspendiert, mit 2 bis 6 Äquivalenten eines Alkalimetallmethylats in überschüssigem Methanol zusammengebracht und anschliessend mit einem Halogenierungsmittel bei Temperaturen zwischen −100 und −50°C umgesetzt und die Reaktion nach erfolgter Halogenierung durch Säurezusatz abgebrochen wird.

6. Verfahren gemäss Anspruch 1, dadurch gekennzeichnet, dass ein Alkalisalz der Cephalosporansäure der allgemeinen Formel I, I', I'' oder I''' mit einem Pivaloyloxymethylhalogenid oder Halogenmethylacetat, Halogenmethylpropionat oder Halogenäthylacetat zu den entsprechenden Estern umgesetzt wird.

**Claims for the Contracting States:** BE, DE, FR, GB, IT, LU, NL, SE, CH/LI

1. Cephalosporins of general formula I:

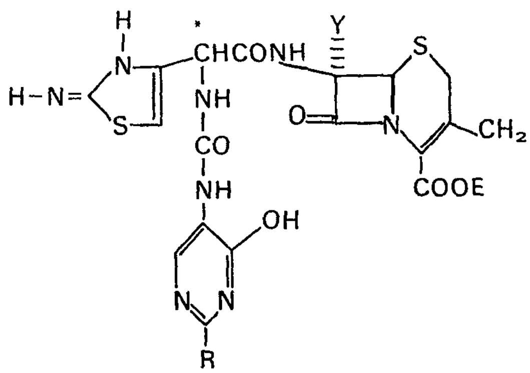
(I)

or the tautomers thereof of general formulae I', I'' and I''':

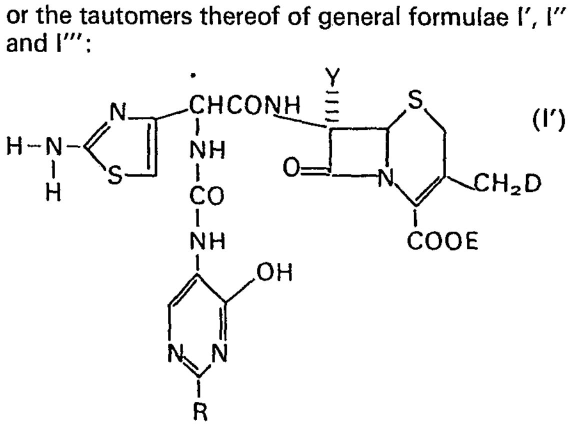
(I')

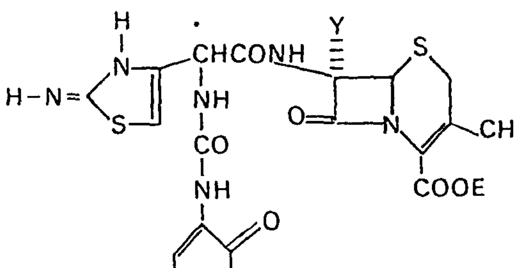
(I'')

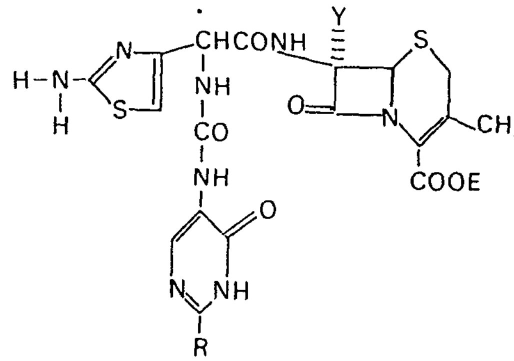
(I''')

wherein:

Y, which is in the α configuration, represents a hydrogen atom or a methoxy group,

D represents an acetoxy group or a group SHet, wherein Het represents a 1-methyltetrazol-5-yl or 2-methyl-1,3,4-thiadiazol-5-yl group, and

E represents a hydrogen atom or an alkali metal or alkaline earth metal ion or a protecting group which can easily be split off *in vitro* or *in vivo*, and R has the following meanings: a cyclopropyl group or a group of general formula $-NHR_1$ wherein $R_1$ represents a methyl, ethyl, propyl, isopropyl, cyclopentyl or cyclohexyl group, or R represents a group of general formula $-NH-Z-X$ wherein $-NH-Z-$ represents a group of formula:

$$-\underset{\displaystyle H}{\underset{|}{N}}-CH_2-CH_2- \text{ or } -\underset{\displaystyle H}{\underset{|}{N}}-(CH_2)_3-$$

and

X represents a hydroxy, methoxy, aminocarbonyl or aminosulphonyl group or wherein

$$-\underset{\displaystyle H}{\underset{|}{N}}-Z-X$$

represents a 4'-hydroxycyclohexylamino group, R may also represent a group of general formula:

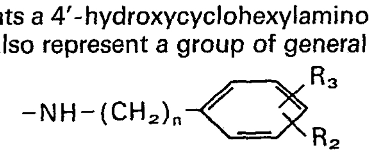

wherein n=0 or 1 and one or two of the groups $R_2$ and $R_3$ represent hydrogen atoms, chlorine atoms, methyl, hydroxy, acetylamino, methylsulphinyl, methylsulphonyl, aminocarbonyl, methylaminocarbonyl, aminosulphonyl, methylaminosulphonyl, methylaminosulphonyl or dimethylaminosulphonyl groups or aminocarbonylamino groups, and the physiologically acceptable salts thereof with inorganic or organic bases.

2. Cephalosporins of general formulae I, I', I'' and I''', as claimed in claim 1, wherein Y, D and E are defined as in claim 1 and R represents a p-aminosulphonylanilino, p-methylsulphinylanilino, p-methylsulphonylanilino, m-hydroxy-p-aminosulphonylanilino, m,p-bis(aminocarbonyl)anilino, p-aminosulphonylbenzylamino, m,p-dihydroxybenzylamino or p-hydroxybenzylamino group.

3. Cephalosporins of general formulae I, I', I'' and I''' as claimed in claims 1 and 2, characterised in that E, being a protecting group which is easily

split off *in vitro* or *in vivo*, represents the benzyl, diphenylmethyl, trityl, t-butyl, 2,2,2-trichloroethyl or trimethylsilyl group, an alkanoyloxyalkyl group with 1 to 5 carbon atoms in the alkanoyl group and 1 to 3 carbon atoms in the alkylene group or a phthalidyl group.

4. Cephalosporins of general formulae I, I', I'' and I''' as claimed in claim 3, characterised in that E represents an acetoxymethyl, propionyloxymethyl, 2-acetoxyethyl or pivaloyloxymethyl group.

5. 7-{D,L-α[(2-p-Aminosulphonylanilino-4-hydroxy-5-pyrimidinyl)ureido]-(2,3-dihydro-2-imino-4-thiazolyl)acetamido}-3-[(1-methyltetrazol-5-yl)thiomethyl]ceph-3-em-4-carboxylic acid and the physiologically acceptable salts thereof with inorganic or organic bases.

6. Process for preparing cephalosporins of general formula I or the tautomers thereof of general formulae I', I'' and I''' as claimed in claim 1 wherein D, E, Y and R are defined as in claim 1 and, if E represents a hydrogen atom, the salts thereof with inorganic or organic bases, characterised in that:

a) a 7-aminocephalosporanic acid derivative of general formula II:

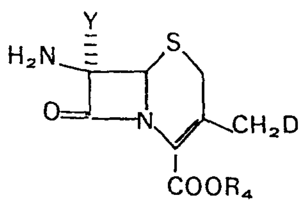

(II)

wherein D is as hereinbefore defined, and $R_4$ represents a tert.-butyl, benzyl, diphenylmethyl, trimethylsilyl or 2,2,2-trichloro-ethyl group, is reacted with a ureidocarboxylic acid of general formula III:

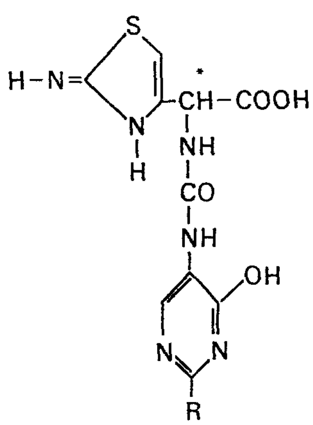

(III)

wherein R is as hereinbefore defined, or with the salts or reactive derivates thereof in a solvent at temperatures of between −40 and +40° C to yield an intermediate product of general formula IV:

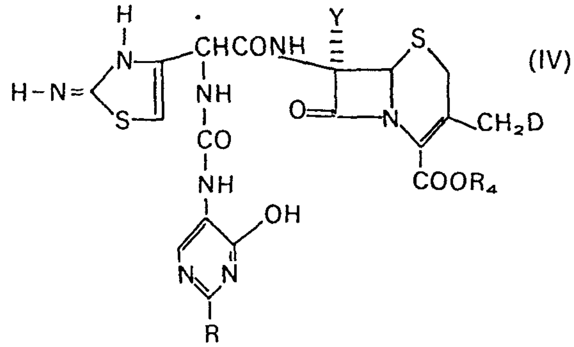

(IV)

and subsequently the group $R_4$ is hydrolytically cleaved from the latter, thus yielding an end product of general formulae I, I', I'' and I''' wherein E represents a hydrogen atom, or

b) a 7-aminocephalosporanic acid of general formula V:

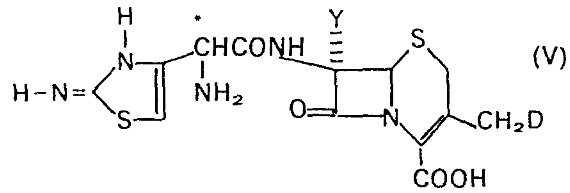

(V)

wherein D and Y are as hereinbefore defined or a salt thereof is reacted with a pyrimidine derivative of general formula VI or VIa:

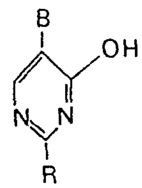

(VI)

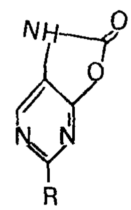

(VIa)

whilist in general formula VI R is as hereinbefore defined and B represents the group −NCO or a reactive derivative of the group −NHCOOH, the group −NHCOCl, −NHCOBr or

−NH−COO− 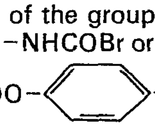 −$NO_2$,

or with mixtures of pyrimidine derivatives wherein B is defined partly according to the first and partly according to the second of the above meanings, in a solvent and within a pH range of between 2.0 and 9.0 at temperatures of between −20 and +50° C, or

c) in order to prepare compounds of general formula I, I', I'' or I''' wherein D represents the group SHet with the meanings for Het given above and E represents hydrogen, a compound of general formula VII:

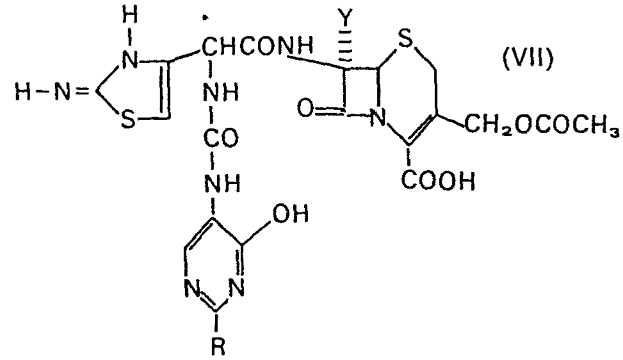

(VII)

wherein R and Y are as hereinbefore defined, is reacted with a compound of general formula VIII:

$$\text{Het-S-M} \qquad \text{(VIII)}$$

wherein Het is as hereinbefore defined and M represents a hydrogen atom or an alkali metal or alkaline earth metal atom, in a solvent at temperatures of between 0 and 100° C in a pH range of from 2 to 10 to yield a compound of general formula I, I', I'' or I''' wherein D represents a group –SHet and E represents a hydrogen atom, or

d) in order to prepare a compound of general formula I, I', I'' or I''' wherein Y represents a methoxy group, a compound of general formula I, I', I'' or I''' wherein Y represents a hydrogen atom is reacted in the presence of methanol with an alkali metal methoxide of general formula $M^+OCH_3^-$, wherein $M^+$ represents an alkali metal ion, and then with a halogenating agent in an inert solvent at temperatures of between −120 and −10° C and, subsequently, if desired, a compound of general formula I, I', I'' or I''' thus obtained, wherein E represents a protecting group which is easily split off *in vitro* or *in vivo* is converted into the free carboxylic acid of general formula I, I', I'' or I''' wherein E represents a hydrogen atom, and/or a compound of general formula I, I', I'' or I''' wherein E represents a hydrogen atom is converted into the esters thereof or into the corresponding salts thereof with inorganic or organic bases.

7. Process as claimed in claim 6a, characterised in that the reactive derivatives of the ureidocarboxylic acids of general formula III used are the acid anhydrides, reactive esters, reactive amides, acid halides or acid azides thereof and the 7-aminocephalosporanic acid derivatives of general formula II used are the compounds wherein E has the meanings given hereinbefore, with the exception of the hydrogen atom, and the reaction is carried out in a solvent, optionally in the presence of a base and/or a condensing agent.

8. Process as claimed in claim 6b, characterised in that a compound of general formula V or a salt thereof with inorganic or organic bases is reacted with a compound of general formula VI or VIa:

a) in water or in water-miscible solvents in the presence of water in a pH range of from 6.5 to 8.0, or

b) in anhydrous solvents, or

c) in a mixture of water and water-immiscible solvents in a pH range of between 6.5 and 8.0

or in that a compound of general formula V wherein the hydrogen atom of the carboxyl group is replaced by a silyl group or another protecting group which is easily split off is reacted with a compound of general formula VI or VIa in a solvent which is free from water and hydroxyl groups or aprotic, optionally in the presence of a base.

9. Pharmaceutical compositions containing one or more compounds of general formulae I, I', I'' and I''' as claimed in claims 1 to 5 together with one or more inert carriers and/or excipients.

10. Use of the cephalosporins of general formulae I, I', I'' and I''' as claimed in claims 1 to 5 for the preparation of pharmaceutical compositions by a non-chemical method.

**Claims for the contracting State:** AT

1. Process for preparing cephalosporins of general formula I:

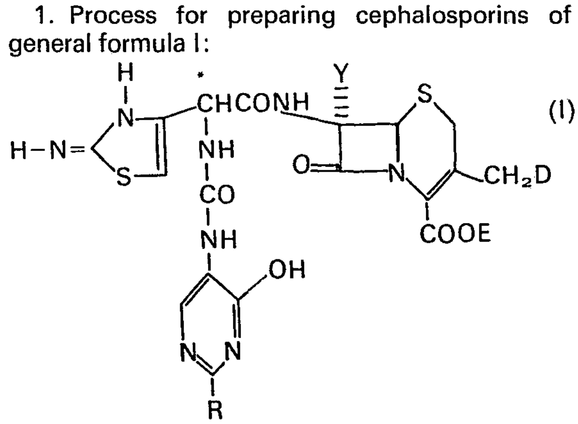

(I)

or the tautomers thereof of general formulae I', I'' and I''':

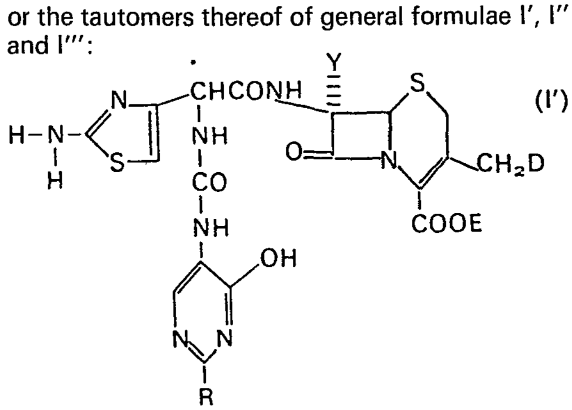

(I')

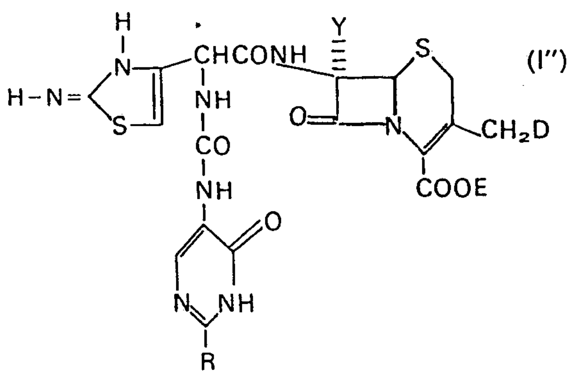

(I'')

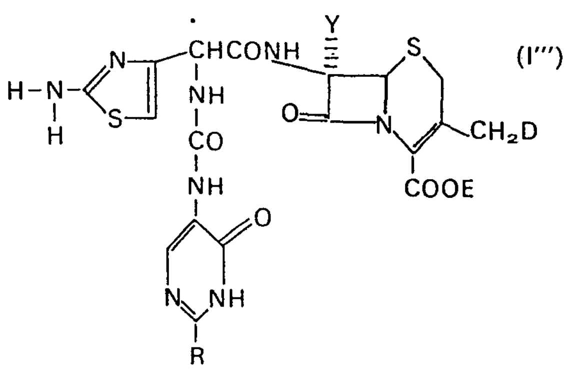

(I''')

wherein:

Y, which is in the α configuration, represents a hydrogen atom or a methoxy group,

D represents an acetoxy group or a group SHet, wherein Het represents a 1-methyltetrazol-5-yl or 2-methyl-1,3,4-thiadiazol-5-yl group, and

E represents a hydrogen atom or an alkali metal or alkaline earth metal ion or a protecting group which can easily be split off *in vitro* or *in vivo*, and R has the following meanings: a cyclopropyl group or a group of general formula $-NHR_1$ wherein $R_1$ represents a methyl, ethyl, propyl, isopropyl, cyclopentyl or cyclohexyl group, or R represents a group of general formula $-NH-Z-X$ wherein $-NH-Z-$ represents a group of formula

$$-\underset{H}{\underset{|}{N}}-CH_2-CH_2- \text{ or } -\underset{H}{\underset{|}{N}}-(CH_2)_3-$$

and

X represents a hydroxy, methoxy, aminocarbonyl or aminosulfonyl group or wherein

$$-\underset{H}{\underset{|}{N}}-Z-X$$

represents a 4'-hydroxycyclohexylamino group, R may also represent a group of general formula:

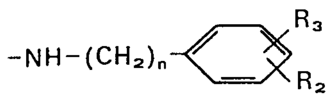

wherein n=0 or 1 and one or two of the groups $R_2$ and $R_3$ represent hydrogen atoms, chlorine atoms, methyl, hydroxy, acetylamino, methylsulphinyl, methylsulphonyl, aminocarbonyl, methylaminocarbonyl, aminosulphonyl, methylaminosulphonyl or dimethylaminosulphonyl groups or aminocarbonylamino groups, and the physiologically acceptable salts thereof with inorganic or organic bases, characterised in that

a) a 7-aminocephalosporanic acid derivative of general formula II:

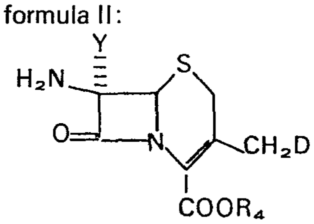

(II)

wherein D is as hereinbefore defined, and $R_4$ represents a tert.-butyl, benzyl, diphenylmethyl, trimethylsilyl or 2,2,2-trichloro-ethyl group, is reacted with a ureidocarboxylic acid of general formula III:

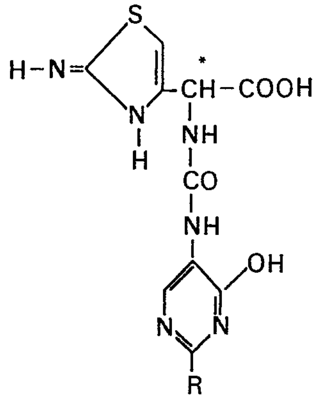

(III)

wherein R is as hereinbefore defined, or with the salts or reactive derivatives thereof in a solvent at temperatures of between −40 and +40°C to yield an intermediate product of general formula IV:

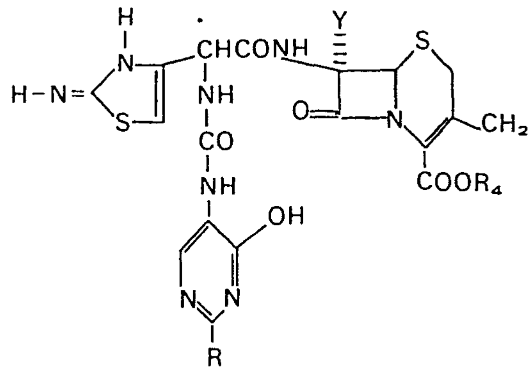

(IV)

and subsequently the group $R_4$ is hydrolytically cleaved from the latter, thus yielding an end product of general formula I, I', I'' and I''' wherein E represents a hydrogen atom, or

b) a 7-aminocephalosporanic acid of general formula V:

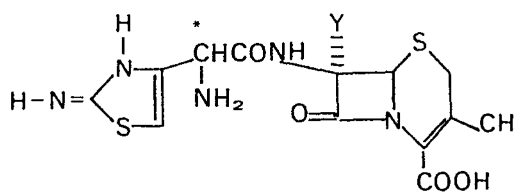

(V)

wherein D and Y are as hereinbefore defined or a salt thereof is reacted with a pyrimidine derivative of general formula VI or VIa:

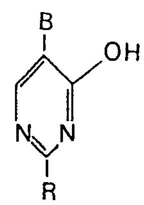

(VI)

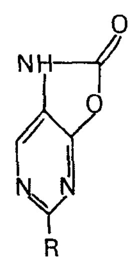

(VIa)

whilst in general formula VI R is as hereinbefore defined and B represents the group −NCO or a reactive derivative of the group −NHCOOH, the group −NHCOCl, −NHCOBr or

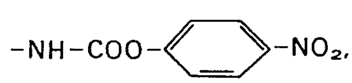

or with mixtures of pyrimidine derivatives wherein B is defined partly according to the first and partly according to the second of the above meanings, in a solvent and within a pH range of between 2.0 and 9.0 at temperatures of between −20 and +50°C, or

c) in order to prepare compounds of general formula I, I', I'' or I''' wherein D represents the group SHet with the meanings for Het given above and E represents hydrogen, a compound of general formula VII:

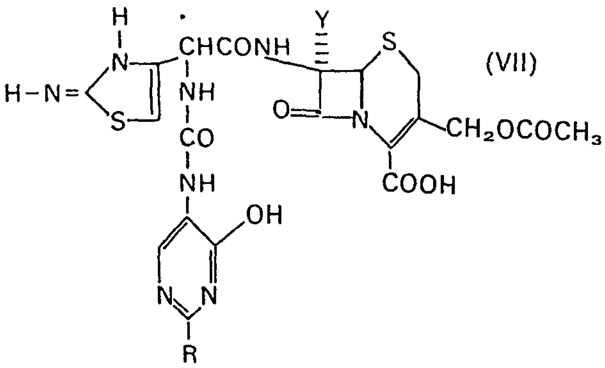

(VII)

wherein R and Y are as hereinbefore defined, is reacted with a compound of general formula VIII:

$$\text{Het-S-M} \qquad \text{(VIII)}$$

wherein Het is as hereinbefore defined and M represents a hydrogen atom or an alkali metal or alkaline earth metal atom, in a solvent at temperatures of between 0 and 100°C in a pH range of from 2 to 10 to yield a compound of general formula I, I', I'' or I''' wherein D represents a group −SHet and E represents a hydrogen atom, or

d) in order to prepare a compound of general formula I, I', I'' or I''' wherein Y represents a methoxy group, a compound of general formula I, I', I'' or I''' wherein Y represents a hydrogen atom is reacted in the presence of methanol with an alkali metal methoxide of general formula $M^+OCH_3^-$, wherein $M^+$ represents an alkali metal ion, and then with a halogenating agent in an inert solvent at temperatures of between −120 and −10°C and, subsequently, if desired, a compound of general formula I, I', I'' or I''' thus obtained, wherein E represents a protecting group which is easily split off *in vitro* or *in vivo* is converted into the free carboxylic acid of general formula I, I', I'' or I''' wherein E represents a hydrogen atom, and/or a compound of general formula I, I', I'' or I''' wherein E represents a hydrogen atom is converted into the esters thereof or into the corresponding salts thereof with inorganic or organic bases.

2. Process as claimed in claim 1a, characterised in that the reactive derivatives of the ureidocarboxylic acids of general formula III used are the acid anhydrides, reactive esters, reactive amides, acid halides or acid azides thereof and the 7-aminocephalosporanic acid derivatives of general formula II used are the compounds wherein E has the meanings given hereinbefore, with the exception of the hydrogen atom, and the reaction is carried out in a solvent, optionally in the presence of a base and/or a condensing agent.

3. Process as claimed in claim 1b, characterised in that a compound of general formula V or a salt thereof with inorganic or organic bases is reacted with a compound of general formula VI or VIa:

a) in water or in water-miscible solvents in the presence of water in a pH range of from 6.5 to 8.0, or

b) in anhydrous solvents, or

c) in a mixture of water and water-immiscible solvents in a pH range of between 6.5 and 8.0 or in that a compound of general formula V wherein the hydrogen atom of the carboxyl group is replaced by a silyl group or another protecting group which is easily split off is reacted with a compound of general formula VI or VIa in a solvent which is free from water and hydroxyl groups or aprotic, optionally in the presence of a base.

4. Process as claimed in claim 1c, characterised in that the reaction is carried out in a strongly polar solvent at a pH of from 4 to 8.

5. Process as claimed in claim 1d, characterised in that a compound of general formula I, I', I''or I''' wherein Y represents a hydrogen atom is dissolved or suspended in an inert agent, combined with 2 to 6 equivalents of an alkali metal methoxide in excess methanol and subsequently reacted with a halogenating agent at temperatures of between −100 and −50°C and after halogenation has been effected the reaction is stopped by the addition of acid.

6. Process as claimed in claim 1, characterised in that an alkali metal salt of cephalosporanic acid of general formula I, I',, I'' or I''' is reacted with a pivaloyloxymethyl halide or halomethyl acetate, halomethyl propionate or halo-ethyl acetate to form the corresponding esters.

**Revendications pour les Etats contractants:** BE, DE, FR, GB, IT, LU, NL, SE, CH

1. Cephalosporines de formule générale I:

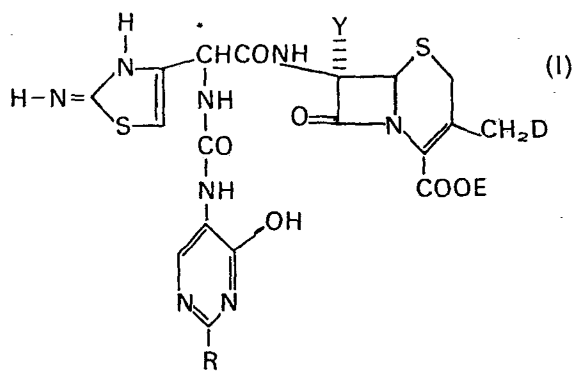

(I)

ou respectivement leurs tautomères de formule générale I', I'' et I''':

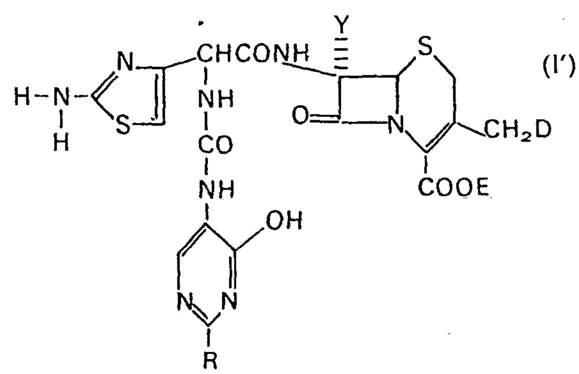

(I')

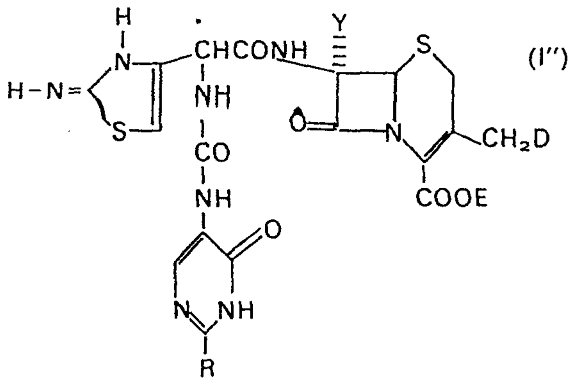

(I'')

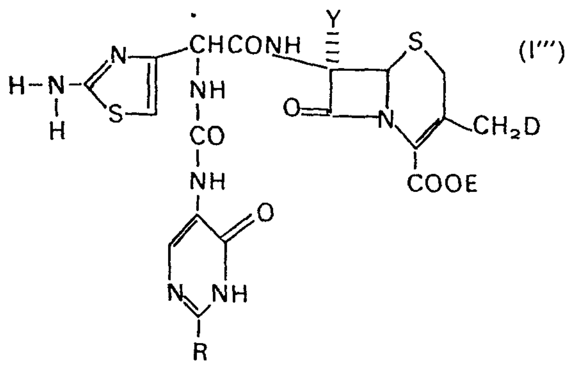

(I''')

où:

Y, qui est dans la configuration α, représente un atome d'hydrogène ou le groupe méthoxy,

D représente le groupe acétoxy ou le groupe SHet, Het représentant le groupe 1-méthyltétrazol-5-yle ou 2-méthyl-1,3,4-thiadiazol-5-yle, et

E représente un atome d'hydrogène ou un ion de métal alcalin ou alcalino-terreux ou un groupe protecteur facilement clivable *in vitro* ou *in vivo*, et

R représente le groupe cyclopropyle ou un groupe de formule générale $-NHR_1$, dans laquelle $R_1$ représente le groupe méthyle, éthyle, propyle, isopropyle, cyclopentyle ou cyclohexyle, ou

R représente un groupe de formule générale $-NH-Z-X$ dans laquelle $-NH-Z-$ représente un groupe de formule

$$-\underset{H}{\underset{|}{N}}-CH_2-CH_2- \text{ ou } -\underset{H}{\underset{|}{N}}-(CH_2)_3-$$

et

X représente le groupe hydroxy, méthoxy, aminocarbonyle ou aminosulfonyle, ou dans laquelle

$$-\underset{H}{\underset{|}{N}}-Z-X$$

représente le groupe 4'-hydroxycyclohexylamino, R peut également représenter un groupe de formule générale:

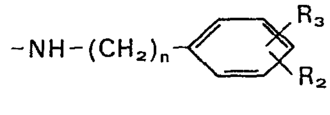

dans laquelle n=0 ou 1 et l'un ou les deux radicaux $R_2$ et $R_3$ représentent des atomes d'hydrogène, des atomes de chlore, des groupes méthyle, hydroxy, acétylamino, méthylsulfinyle, méthylsulfonyle, aminocarbonyle, méthylaminocarbonyle, aminosulfonyle, méthylaminosulfonyle ou diméthylaminosulfonyle ou des groupes aminocarbonylamino, et leurs sels physiologiquement supportables avec des bases minérales ou organiques.

2. Cephalosporines de formules générales I, I', I'' et I''' selon la revendication 1, dans lesquelles Y, D et E sont définis comme indiqué dans la revendication 1, et R représente un groupe p-aminosulfonylanilino, p-méthylsulfinylanilino, p-méthylsulfonylanilino, m-hydroxy-p-aminosulfonylanilino, m,p-bis-(aminocarbonyl)anilino, p-aminosulfonylbenzylamino, m,p-dihydroxybenzylamino ou p-hydroxybenzylamino.

3. Cephalosporines de formules générales I, I', I'' et I''' selon les revendications 1 et 2, caractérisées en ce que E, en tant que groupe facilement clivable *in vitro* ou *in vivo*, représente le groupe benzyle, diphénylméthyle, trityle, t-butyle, 2,2,2-trichloroéthyle ou triméthylsilyle, un groupe alcanoyloxyalcoyle avec 1 à 5 atomes de carbone dans le radical alcanoyle et 1 à 3 atomes de carbone dans le radical alcoylène ou le groupe phtalidyle.

4. Cephalosporines de formules générales I, I', I'' et I''' selon la revendication 3, caractérisées en ce que E représente le groupe acétoxyméthyle, propionyloxyméthyle, 2-acétoxyéthyle ou pivaloyloxyméthyle.

5. L'acide 7-{D,L-α-[(2-p-aminosulfonylanilino-4-hydroxy-5-pyrimidinyl)uréido]-(2,3-dihydro-2-imino-4-thiazolyl)acétamido}-3-[(1-méthyltétrazol-5-yl)thiométhyl]céph-3-ème-4-carboxylique et ses sels physiologiquement supportables avec des bases minérales ou organiques.

6. Procédé pour la préparation de céphalosporines de formule générale I ou respectivement de leurs tautomères de formules générales I', I'' et I''' selon la revendication 1, où D, E, Y et R sont définis comme indiqué dans la revendication 1, et, lorsque E représente un atome d'hydrogène, de leurs sels avec des bases minérales ou organiques, caractérisé en ce que:

a) on fait réagir un dérivé d'acide 7-aminocéphalosporanique de formule générale II:

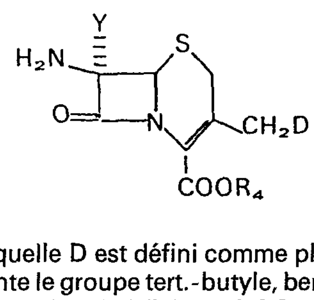

(II)

dans laquelle D est défini comme plus haut, et $R_4$ représente le groupe tert.-butyle, benzyle, diphénylméthyle, triméthylsilyle ou 2,2,2-trichloroéthyle, avec un acide uréidocarboxylique de formule générale III:

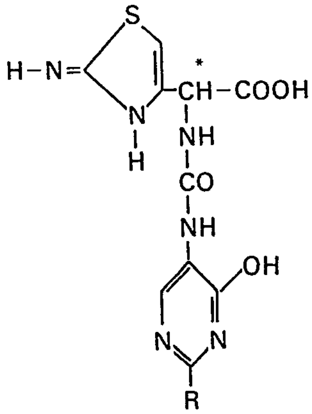

(III)

dans laquelle R possède les significations mentionnées plus haut, ou avec ses sels ou dérivés réactifs dans un solvant à des températures entre −40 et +40°C pour obtenir un produit intermédiaire de formule générale IV:

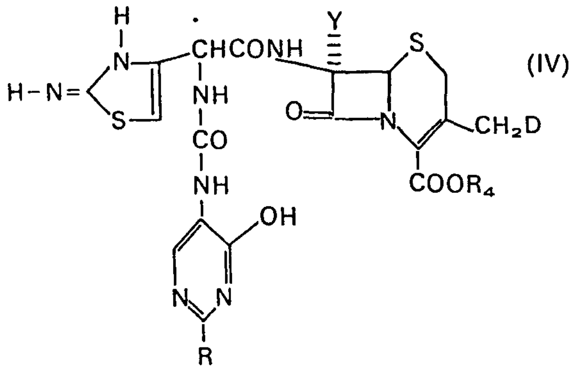

(IV)

et en ce que, à partir de ce dernier, on clive ensuite hydrolytiquement le radical $R_4$, le produit final de formules générales I, I', I'' et I''' en résultant, dans lequel E représente un atome d'hydrogène, ou

b) on fait réagir un acide 7-aminocéphalosporanique de formule générale V:

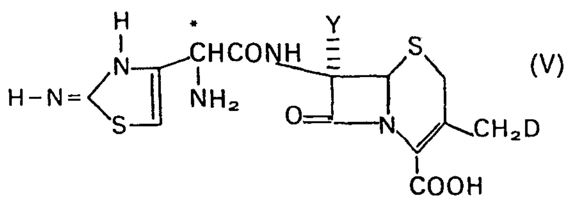

(V)

dans laquelle D et Y sont définis comme plus haut ou ses sels avec un dérivé de pyrimidine de formule générale VI et VIa:

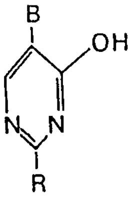

(VI)

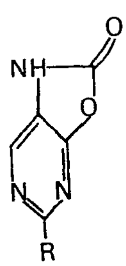

(VIa)

dans la formule générale VI R étant défini comme plus haut et B représentant le groupe −NCO ou un dérivé réactif du groupe −NHCOOH, le groupe −NHCOCl, −NHCOBr ou

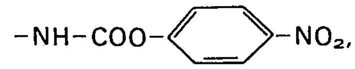

ou avec des mélanges de tels dérivés de pyrimidine dans lesquels B possède en partie l'une et en partie les autres des significations mentionnées plus haut, dans un solvant et dans un domaine de pH entre 2,0 et 9,0 à des températures entre −20 et +50°C, ou

c) pour la préparation de composés de formule générale I, I', I'' ou I''', dans laquelle D représente le groupe SHet avec les significations indiquées plus haut pour Het et E représente l'hydrogène, on fait réagir un composé de formule générale VII:

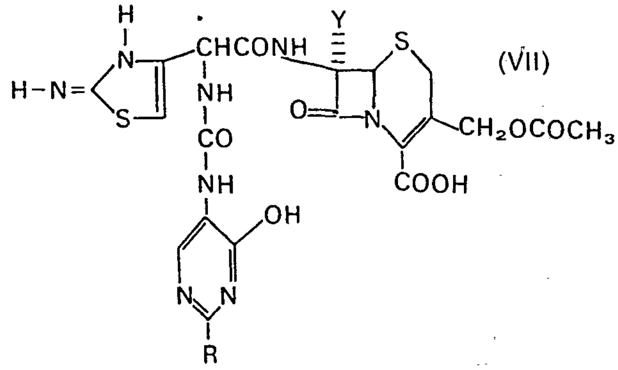

(VII)

dans laquelle R et Y ont les significations indiquées plus haut, avec un composé de formule générale VIII:

$$Het{-}S{-}M \qquad (VIII)$$

dans laquelle Het a les significations indiquées plus haut et M représente un atome d'hydrogène ou un ion de métal alcalin ou de métal alcalino-terreux, dans un solvant à des températures entre 0 et 100°C, dans un domaine de pH entre 2 et 10, pour donner un composé de formule générale I, I', I'' ou I''', dans laquelle D possède les significations d'un groupe −SHet et E celle d'un atome d'hydrogène, ou

d) pour la préparation d'un composé de formule générale I, I', I'' ou I''' dans laquelle Y représente le groupe méthoxy, on fait réagir un composé de formule générale I, I', I'' ou I''' dans laquelle Y représente un atome d'hydrogène, en présence de méthanol avec un méthylate de métal alcalin de formule générale $M^+OCH_3^-$, où $M^+$ représente un ion de métal alcalin, puis avec un agent d'halogénation dans un solvant inerte à des températures entre −120 et −10°C, et, si on le désire, on transforme ensuite un composé ainsi obtenu de formule générale I, I', I'' ou I''' où E représente un groupe protecteur facilement clivable *in vitro* ou *in vivo*, en l'acide carboxylique libre de formule générale I, I', I'' ou I''' dans laquelle E est un atome d'hydrogène, et/ou on transforme un composé de formule générale I, I', I'' ou I''' dans laquelle E représente un atome d'hydrogène en ses esters ou, au moyen de bases minérales ou organiques, en les sels correspondants.

7. Procédé selon la revendication 6a, caractérisé en ce que l'on utilise, en tant que dérivés réactifs des acides uréidocarboxyliques de formule générale III, leurs anhydrides d'acides, leurs esters réactifs, leurs amides réactifs, leurs halogénures d'acides ou azides d'acides et, en tant que dérivés de l'acide 7-aminocéphalosporanique de formule générale II, des composés dans lesquels E possède les significations mentionnées au début, à l'exception de celle d'un atome d'hydrogène, et en ce que la réaction a lieu dans un solvant, éventuellement en présence d'une base et/ou d'un agent de condensation.

8. Procédé selon la revendication 6b, caractérisé en ce qu'on fait réagir un composé de formule générale V ou un sel avec des bases minérales ou organiques avec un composé de formule générale VI ou VIa:

a) dans l'eau ou dans des solvants miscibles à l'eau en présence d'eau dans un domaine de pH de 6,5 à 8,0, ou

b) dans des solvants anhydres, ou

c) dans un mélange d'eau et de solvants non miscibles à l'eau dans un domaine de pH entre 6,5 et 8,0,

ou en ce qu'on fait réagir un composé de formule générale V, dans laquelle l'atome d'hydrogène du groupe carboxyle est remplacé par un groupe silyle ou un autre groupe protecteur facilement clivable, avec un composé de formule générale VI ou VIa dans un solvant ne contenant ni eau ni groupes hydroxy ou aprotique, éventuellement en présence d'une base.

9. Médicament contenant un ou plusieurs composés de formules générales I, I', I'' et I''' selon les revendications 1 à 5 conjointement à un ou plusieurs excipients et/ou adjuvants inertes.

10. Utilisation des céphalosporines de formules générales I, I', I'' et I''' selon les revendications 1 à 5 pour la préparation de médicaments par voie non chimique.

**Revendications pour l'Etat contractant:** AT

1. Procédé pour la préparation de céphalosporines de formule générale I:

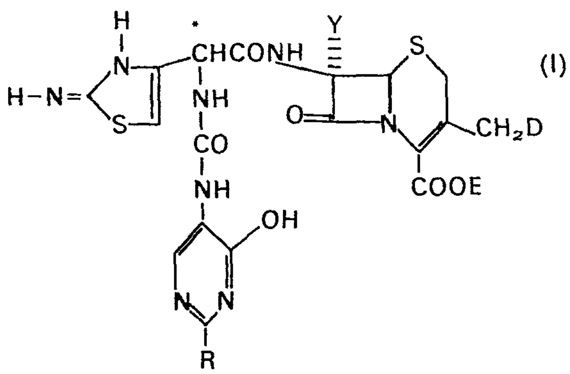

(I)

ou respectivement de leurs tautomères de formule générale I', I'' et I''':

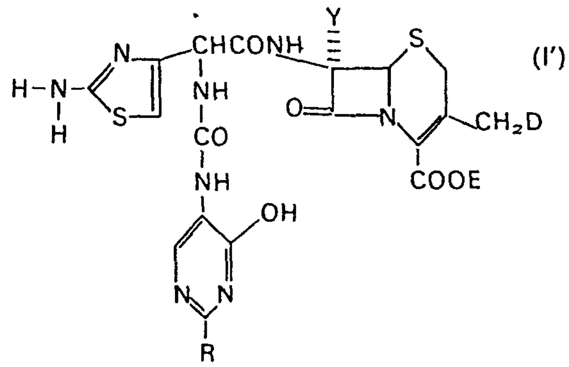

(I')

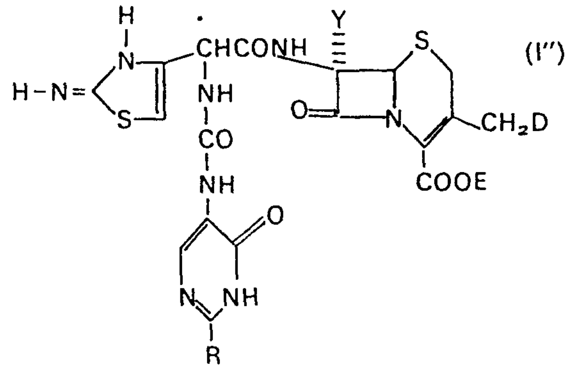

(I'')

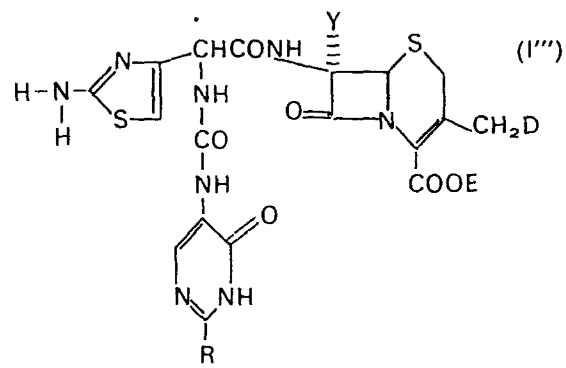

(I''')

où:

Y, qui est dans la configuration α, représente un atome d'hydrogène ou le groupe méthoxy,

D représente le groupe acétoxy ou le groupe SHet, Het représentant le groupe 1-méthyltétrazol-5-yle ou 2-méthyl-1,3,4-thiadiazol-5-yle,

E représente un atome d'hydrogène ou un ion de métal alcalin ou alcalino-terreux ou un groupe protecteur facilement clivable *in vitro* ou *in vivo*, et

R représente le groupe cyclopropyle ou un groupe de formule générale $-NHR_1$, dans laquelle $R_1$ représente le groupe méthyle, éthyle, propyle, isopropyle, cyclopentyle ou cyclohexyle, ou

R représente un groupe de formule générale $-NH-Z-X$ dans laquelle $-NH-Z-$ représente un groupe de formule

$$-\underset{\displaystyle H}{\underset{|}{N}}-CH_2-CH_2- \text{ ou } -\underset{\displaystyle H}{\underset{|}{N}}-(CH_2)_3-$$

et

X représente le groupe hydroxy, méthoxy, aminocarbonyle ou aminosulfonyle, ou dans laquelle

$$-\underset{\displaystyle H}{\underset{|}{N}}-Z-X$$

représente le groupe 4'-hydroxycyclohexylamino, R peut également représenter un groupe de formule générale:

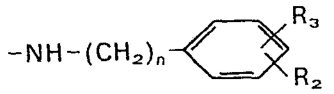

dans laquelle n=0 ou 1 et l'un ou les deux radicaux $R_2$ et $R_3$ représentent des atomes d'hydrogène, des atomes de chlore, des groupes méthyle, hydroxy, acétylamino, méthylsulfinyle, méthylsulfonyle, aminocarbonyle, méthylaminocarbonyle, aminosulfonyle, méthylaminosulfonyle ou diméthylaminosulfonyle ou des groupes aminocarbonylamino, et leurs sels physiologiquement supportables avec des bases minérales ou organiques, caractérisé en ce que:

a) on fait réagir un dérivé d'acide 7-aminocéphalosporanique de formule générale II:

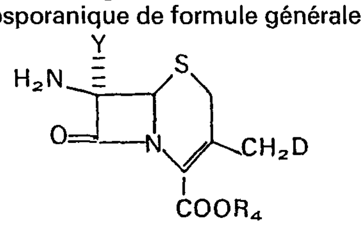

(II)

dans laquelle D est défini comme plus haut, et $R_4$ représente le groupe tert.-butyle, benzyle, diphénylméthyle, triméthylsilyle ou 2,2,2-trichloroéthyle, avec un acide uréidocarboxylique de formule générale III:

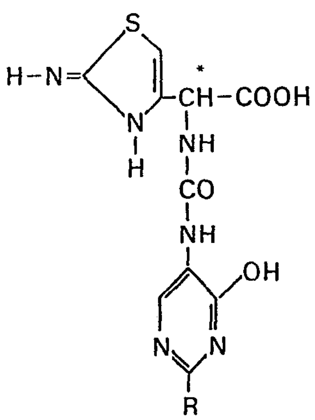

(III)

dans laquelle R possède les significations mentionnées plus haut, ou avec ses sels ou dérivés réactifs dans un solvant à des températures entre −40 et +40°C pour obtenir un produit intermédiaire de formule générale IV:

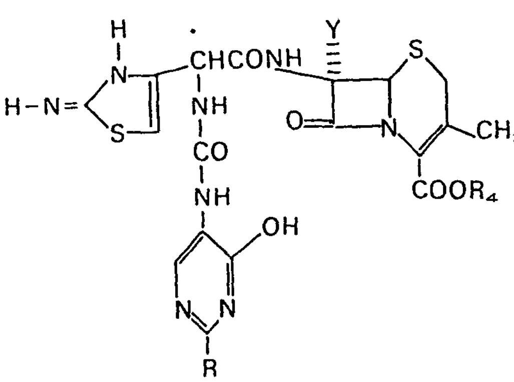

(IV)

et en ce que, à partir de ce dernier, on clive ensuite hydrolytiquement le radical $R_4$, le produit final de formules générales I, I', I'' et I''' en résultant, dans lequel E représente un atome d'hydrogène, ou

b) on fait réagir un acide 7-aminocéphalosporanique de formule générale V:

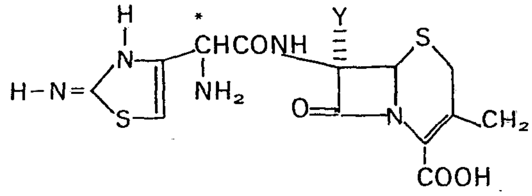

(V)

dans laquelle D et Y sont définis comme plus haut, ou ses sels avec un dérivé de pyrimidine de formule générale VI ou VIa:

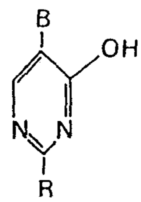

(VI)

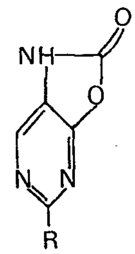

(VIa)

dans la formule générale VI R étant défini comme plus haut et B représentant le groupe −NCO ou un dérivé réactif du groupe −NHCOOH, le groupe −NHCOCl, −NHCOBr ou

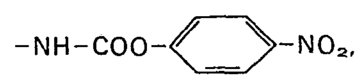

ou avec des mélanges de tels dérivés de pyrimidine dans lesquels B possède en partie l'une et en partie les autres des significations mentionnées plus haut, dans un solvant et dans un domaine de pH entre 2,0 et 9,0 à des températures entre −20 et +50°C, ou

c) pour la préaration de composés de formule générale I, I', I'' ou I''', dans laquelle D représente le groupe SHet avec les significations indiquées plus haut pour Het et E représente l'hydrogène, on fait réagir un composé de formule générale VII:

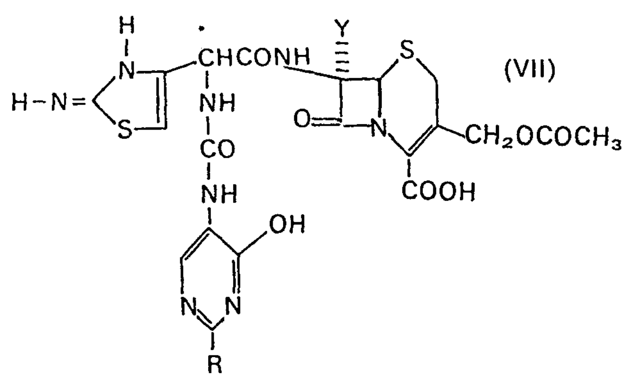

(VII)

dans laquelle R et Y ont les significations indiquées plus haut, avec un composé de formule générale VIII:

Het−S−M (VIII)

dans laquelle Het a les significations indiquées plus haut et M représente un atome d'hydrogène

ou un ion de métal alcalin ou de métal alcalino-terreux, dans un solvant à des températures entre 0 et 100°C, dans un domaine de pH entre 2 et 10, pour donner un composé de formule générale I, I', I" ou I"', dans laquelle D possède les significations d'un groupe −SHet et E celle d'un atome d'hydrogène, ou

d) pour la préparation d'un composé de formule générale I, I', I" ou I"' dans laquelle Y représente le groupe méthoxy, on fait réagir un composé de formule générale I, I', I" ou I"' dans laquelle Y représente un atome d'hydrogène, en présence de méthanol avec un méthylate de métal alcalin de formule générale $M^+OCH_3^-$, où $M^+$ représente un ion de métal alcalin, puis avec un agent d'halogénation dans un solvant inerte à des températures entre −120 et −10°C, et, si on le désire, on transforme ensuite un composé ainsi obtenu de formule générale I, I', I" ou I"' où E représente un groupe protecteur facilement clivable *in vitro* ou *in vivo*, en l'acide carboxylique libre de formule générale I, I', I" ou I"' dans laquelle E est un atome d'hydrogène, et/ou on transforme un composé de formule générale I, I', I" ou I"' dans laquelle E représente un atome d'hydrogène en ses esters ou, au moyen de bases minérales ou organiques, en les sels correspondants.

2. Procédé selon la revendication 1a, caractérisé en ce que l'on utilise, en tant que dérivés réactifs des acides uréidocarboxyliques de formule générale III, leurs anhydrides d'acides, leurs esters réactifs, leurs amides réactifs, leurs halogénures d'acides ou azides d'acides et, en tant que dérivés de l'acide 7-aminocéphalosporanique de formule générale II, des composés dans lesquels E possède les significations mentionnées au début, à l'exception de celle d'un atome d'hydrogène, et en ce que la réaction a lieu dans un solvant, éventuellement en présence d'une base et/ou d'un agent de condensation.

3. Procédé selon la revendication 1b, caractérisé en ce qu'on fait réagir un composé de formule générale V ou un de ses sels avec des bases minérales ou organiques avec un composé de formule générale VI ou VIa:

a) dans l'eau ou dans des solvants miscibles à l'eau en présence d'eau dans un domaine de pH de 6,5 à 8,0, ou

b) dans des solvants anhydres, ou

c) dans un mélange d'eau et de solvants non miscibles à l'eau dans un domaine de pH entre 6,5 et 8,0,

ou en ce qu'on fait réagir un composé de formule générale V, dans laquelle l'atome d'hydrogène du groupe carboxyle est remplacé par un groupe silyle ou un autre groupe protecteur facilement clivable, avec un composé de formule générale VI ou VIa dans un solvant ne contenant ni eau ni groupes hydroxy ou aprotique, éventuellement en présence d'une base.

4. Procédé selon la revendication 1c, caractérisé en ce que la réaction est effectuée dans un solvant fortement polaire à une valeur de pH de 4 à 8.

5. Procédé selon la revendication 1d, caractérisé en ce qu'on met ensemble un composé de formule générale I, I', I" ou I"' où Y représente un atome d'hydrogène, dissous ou en suspension dans un agent inerte, avec 2 à 6 équivalents d'un méthylate de métal alcalin dans du méthanol en excès, et en ce qu'on fait réagir ensuite avec un agent d'halogénation à des températures entre −100 et −50°C, et en ce qu'on arrête la réaction après que l'halogénation a eu lieu, par addition d'acide.

6. Procédé selon la revendication 1, caractérisé en ce que l'on fait réagir un sel alcalin de l'acide céphalosporanique de formule générale I, I', I" ou I"' avec un halogénure de pivaloyloxyméthyle ou un acétate d'halogénométhyle, un propionate d'halogénométhyle ou un acétate d'halogénoéthyle pour donner les esters correspondants.